# EUROPEAN PATENT APPLICATION

(11) **EP 4 656 153 A1**
(43) Date of publication of application: **03.12.2025**
(21) Application number: 25179982.1
(22) Date of filing: 30.05.2025
(51) Int. Cl.: A61B 34/10, A61B 34/00

(54) **SYSTEM AND METHOD FOR SURGICAL PLANNING AND ASSESSMENT OF PATIENT ANATOMY WITH MOTION DATA**

(30) Priority: 31.05.2024 US 202463654585 P
(71) Applicant: Stryker Corporation, Portage, MI 49002 (US)
(72) Inventor: LAMBERS, Floor, Portage, 49002 (US); HÖTZEL, Linus, Portage, 49002 (US); GOTTSCHLING, Heiko, Portage, 49002 (US); SCHRÖDER, Manuel, Portage, 49002 (US)
(74) Representative: V.O.

(57) **Abstract**

Methods of assessing a patient shoulder anatomy is provided. The method includes receiving one or more 3D models based on the patient shoulder anatomy, applying motion data based on a glenohumeral joint to the one or more 3D models, and determining a track engagement of the glenohumeral joint based on the applied motion data. A computing system programmed to perform these methods is also described.

## Description

### CROSS REFERENCE TO RELATED APPLICATIONS

This application claims priority to U.S. Provisional Application No. 63/654,585, filed on May 31, 2024, which is hereby incorporated by reference in its entirety.

### BACKGROUND

The glenohumeral joint is highly mobile and susceptible to dislocation. When healthy, the humerus is attached to the glenoid by the muscles and tendons of the rotator cuff, padded by soft tissue, and freely rotates relative to the glenoid. A healthy glenohumeral joint allows the shoulder to have a large and comfortable range of motion. However, shoulder dislocation is a common injury that results in the removal of the humerus from the glenoid. After one occurrence of a shoulder dislocation, subsequent dislocation becomes more likely due to increased instability of the glenohumeral joint. This damage may be in the form of a lesion on the humeral head or a lesion on the glenoid and impacts the engagement between the humerus and the glenoid, leading to joint instability. Surgical procedures exist to resolve these lesions, but surgical planning must be completed to determine which procedures may be suitable. Therefore, some assessment of the glenohumeral joint must be completed to determine the condition of the shoulder and what prospective surgeries may be recommended.

An assessment of the shoulder anatomy may inform the likelihood of future dislocation of the glenohumeral joint. Depending on this assessment, surgeons can generate a surgical plan according to the needs of the patient, which could include soft tissue repair, bone grafting, capsular plication, capsular shift, or joint replacement. Without this assessment, surgeons have very little guidance for decision making to restore the health of the patient glenohumeral joint. To complete an assessment, surgeons may visualize the patient shoulder anatomy and complete an instability analysis of the joint. Additionally, surgeons may study kinematic motions of the shoulder joint to determine the state of the anatomy and study differences between an injured patient and a healthy model. A 3D model of the joint may be generated from image data captured by an imaging system and may be manipulated by a surgeon to provide information to evaluate the state of the patient shoulder anatomy. Current practices are time-intensive, laborious, and prone to inconsistency.

### SUMMARY

Features and advantages of the present invention will be readily appreciated as the same becomes better understood after reading the subsequent description taken in connection with the accompanying drawings.

According to a first aspect, a method of assessing a patient shoulder anatomy is provided. The method includes receiving one or more 3D models based on the patient shoulder anatomy, applying motion data based on a glenohumeral joint to the one or more 3D models, and determining a track engagement of the glenohumeral joint based on the applied motion data.

According to a second aspect, a method of visualizing a patient shoulder anatomy is provided. The method includes receiving one or more 3D models of the patient shoulder anatomy which include a scapula 3D model and a humerus 3D model. The method also includes applying kinematic data of a scapula and a humerus to the one or more 3D models, the kinematic data including one or more translation trajectories of the scapula and the humerus relative to one another and one or more rotation trajectories of the scapula and the humerus relative to one another. The method also includes generating a global coordinate system, displaying renderings of a first pose of the scapula model and the humerus model relative to the global coordinate system, and displaying renderings of a second pose of the scapula model and the humerus model relative to the global coordinate system and differing from the first pose in at least one translational degree of freedom and one rotational degree of freedom.

According to a third aspect, a method of assessing a patient shoulder anatomy is provided. The method includes receiving 3D models of a first bone and a second bone which interact to form a joint. The method also includes applying motion data to the joint to move the first bone model and the second bone model relative to one another. The motion data may include a first pose of the first bone relative to the second bone and a second pose of the first bone relative to the second bone, where the first pose differs from the second pose in at least one translational degree of freedom and one rotational degree of freedom. The method also includes determining a joint characteristic of the joint based on the poses of the first 3D bone model and the second 3D bone model as a result of the applied motion data.

According to a fourth aspect, a method of visualizing a patient shoulder anatomy is provided that includes receiving image data of the patient shoulder anatomy including at least a portion of soft tissue, a humerus, and a scapula. The method also includes generating a humerus 3d model and a scapula 3d model based on a segmentation of the image data. The method also includes determining a location of at least one insertion point of the soft tissue corresponding to an attachment of soft tissue to the humerus and scapula. The method also includes generating an insertion virtual object based on the at least one insertion point of the soft tissue. The method also includes generating a terminal position of the humerus 3d model relative to the scapula 3d model. The method also includes determining a glenoid projection virtual object based on the terminal position of the humerus 3d model relative to the scapula 3d model. The method also includes displaying: a rendering of the humerus 3d model or the scapula 3d model, a rendering of insertion virtual object, and a rendering of the glenoid projection virtual object. Other embodiments of this aspect include corresponding computer systems, apparatus, and computer programs recorded on one or more computer storage devices, each configured to perform the actions of the methods.

In another general aspect includes a method of visualizing a patient shoulder anatomy. The method also includes receiving image data of the patient shoulder anatomy including least a portion of soft tissue, a humerus, and a scapula. The method also includes generating a humerus 3d model and a scapula 3d model based on a segmentation of the image data. The method also includes generating a terminal position of the humerus 3d model relative to the scapula 3d model. The method also includes determining a glenoid projection virtual object based on the terminal position of the humerus 3d model relative to the scapula 3d model. The method also includes generating a non-patient glenoid projection virtual object based on a terminal position of a humerus relative to a scapula for at least one person other than the patient. The method also includes displaying a rendering of non-patient glenoid projection virtual object and a rendering of the glenoid projection virtual object. Other embodiments of this aspect include corresponding computer systems, apparatus, and computer programs recorded on one or

Any of the above aspects can be combined in part or in whole with any other aspect.

### BRIEF DESCRIPTION OF THE DRAWINGS

Advantages of the invention will be readily appreciated as the same becomes better understood by reference to the following detailed description when considered in connection with the accompanying drawings, wherein:
FIG. 1 is a perspective view of an exemplary layout of an operating room including a surgical planning system, according to one implementation.
FIG. 2 is a screenshot displaying renderings of 3D models of a patient glenoid and humerus that may be shown on a display of the surgical planning system according to one implementation.
FIG. 3A is a screenshot displaying renderings of 3D models of a first pose of forward elevation of a glenohumeral joint after motion data has been applied to the 3D models that may be shown on the display according to one implementation.
FIG. 3B is a screenshot displaying a rendering of the 3D models of FIG. 3A of a terminal pose of forward elevation of a glenohumeral joint after motion data has been applied to the 3D models that may be shown on the display according to one implementation.
FIG. 4A is a screenshot displaying a rendering of the 3D models of a first pose of scapula abduction of a glenohumeral joint after motion data has been applied to the 3D models that may be shown on the display according to one implementation.
FIG. 4B is a screenshot displaying a rendering of the 3D models of FIG. 4A of a terminal pose of scapula abduction of a glenohumeral joint after motion data has been applied to the 3D models that may be shown on the display according to one implementation.
FIG. 5A is a screenshot displaying a rendering of the 3D models of a first pose of coronal abduction of a glenohumeral joint after motion data has been applied to the 3D models that may be shown on the display according to one implementation.
FIG. 5B is a screenshot displaying a rendering of the 3D models of FIG. 5A of a terminal pose of coronal abduction of a glenohumeral joint after motion data has been applied to the 3D models that may be shown on the display according to one implementation.
FIG. 6A is a screenshot displaying a rendering of the 3D models of a first pose of external rotation at 90° of abduction of a glenohumeral joint after motion data has been applied to the 3D models that may be shown on the display according to one implementation.
FIG. 6B is a screenshot displaying a rendering of the 3D models of FIG. 6A of a terminal pose of external rotation at 90° of abduction of a glenohumeral joint after motion data has been applied to the 3D models that may be shown on the display according to one implementation.
FIG. 7A is a screenshot displaying a rendering of the 3D models of a first pose of external rotation at rest of a glenohumeral joint after motion data has been applied to the 3D models that may be shown on the display according to one implementation.
FIG. 7B is a screenshot displaying a rendering of the 3D models of FIG. 7A of a terminal pose of external rotation at rest of a glenohumeral joint after motion data has been applied to the 3D model that may be shown on the display according to one implementation.
FIG. 8 shows an example of scapula translation data associated with coronal abduction that may be applied to a 3D model of the humerus and a 3D model of the scapula, according to one implementation.
FIG. 9 shows an example of scapula rotation data associated with coronal abduction that may be applied to a humerus 3D model and a scapula 3D model, according to one implementation.
FIG. 10 shows how a controller of the surgical planning system of FIG. 1 may be configured to align local coordinate systems of a humerus 3D model and a scapula 3D model, according to one implementation.
FIG. 11 shows how a controller of the surgical planning system of FIG. 1 may be configured to generate local coordinate systems of a humerus 3D model and a scapula 3D model, according to one implementation.
FIG. 12 is a perspective representation of a series of poses of 3D models of a humerus and a scapula after motion data has been applied to the 3D models, according to one implementation.
FIG. 13 shows a series of steps the controller of FIG. 10 may be configured to follow to determine a location of a glenoid track virtual object relative to the humerus 3D model of FIG. 10, according to one implementation.
FIG. 14 shows a series of steps the controller of FIG. 10 may be configured to follow to determine a location of a glenoid track line on the humerus 3D model of FIG. 10, according to one implementation.
FIG. 15 shows how the controller of FIG. 10 may be configured to determine a glenoid center of a 3D model of a scapula, according to one implementation.
FIG. 16 is a screenshot of the rendering of the humerus 3D model of FIG. 10 including a representation of a lesion virtual object relative to a glenoid projection virtual object, according to one implementation.
FIG. 17 shows a screenshot of a rendering of a glenoid outline on a rendering of a scapula 3D model, according to one implementation.
FIG. 18 and 19 are screenshots of renderings of the humerus 3D model of FIG. 10 including a representation of a lesion virtual object relative to a glenoid projection virtual object, according to one implementation.
FIG. 20 shows a step that the controller of FIG. 10 may be configured to follow to determine a non-patient glenoid projection virtual object and display the non-patient glenoid projection object on a rendering of the humerus 3D model according to one embodiment.
FIG. 21 shows four screenshots of the rendering of the humerus 3D model including various representations of a lesion virtual object, a glenoid projection virtual object, a planned glenoid projection virtual object, and/or a non-patent glenoid projection virtual object according to one implementation.
FIG. 22 shows patient image data that includes rotator cuff muscles and will be used to describe how the controller is configured to apply a segmentation algorithm to the patient image data to determine a soft tissue insertion line, according to one implementation.
FIGS. 23 - 24B show patient image data that includes a humerus and will be used to describe how the controller of the surgical planning system of FIG. 1 may be configured to determine the soft tissue insertion line of FIG. 22, according to various implementations.
FIG. 25 shows four screenshots of the rendering of the humerus 3D model including various representations of a lesion virtual object, a glenoid projection virtual object, a planned glenoid projection virtual object, and/or a soft tissue line according to one implementation.
FIG. 26A depicts a humerus including a plurality of radiopaque beads.
FIG. 26B depicts a chart that includes the clockface position of the plurality of radiopaque beads of FIG. 26A for a plurality of non-patient persons against the percentage of humeral width, including an average line.
FIG. 26C depicts representative a humerus with the various clockface positions of FIG. 26B.
FIG. 27 depicts a flowchart illustrating steps of assessing a patient shoulder anatomy by applying motion data to 3D models, according to one implementation.
FIG. 28 depicts a flowchart illustrating steps of visualizing a patient shoulder anatomy by displaying renderings of a scapula 3D model and a humerus 3D model, according to one implementation.
FIG. 29 depicts a flowchart illustrating steps of assessing a patient anatomy based on applying motion data to a first bone 3D model and a second bone 3D model, according to one implementation.
FIG. 30 depicts a flowchart illustrating steps of assessing a patient anatomy that includes generating a rendering of the humerus 3D model, a rendering of an insertion virtual object and a glenoid projection virtual object, according to one implementation.
FIG. 31 depicts a flowchart illustrating steps of assessing a patient anatomy that incudes generating a non-patient glenoid projection virtual object based on a terminal position of a humerus relative to a scapula for at least one non-patient person and displaying a rendering of the non-patient patient glenoid projection virtual object and a rendering of the glenoid projection virtual object of the patient.

### DETAILED DESCRIPTION OF THE INVENTION

### I. Example System Overview

Referring to the Figures wherein like numerals indicate like or corresponding parts throughout the several views, a surgical planning system 100 including a controller 110 and methods for using the same are shown throughout. It is to be understood that some Figures may represent a portion and/or the entirety of a graphical user interface, and the presented views do not limit the graphical user interface to any configuration shown herein. The graphical user interface may include any combination of the Figures and those not specifically shown may still be utilized by the surgical planning system 100.

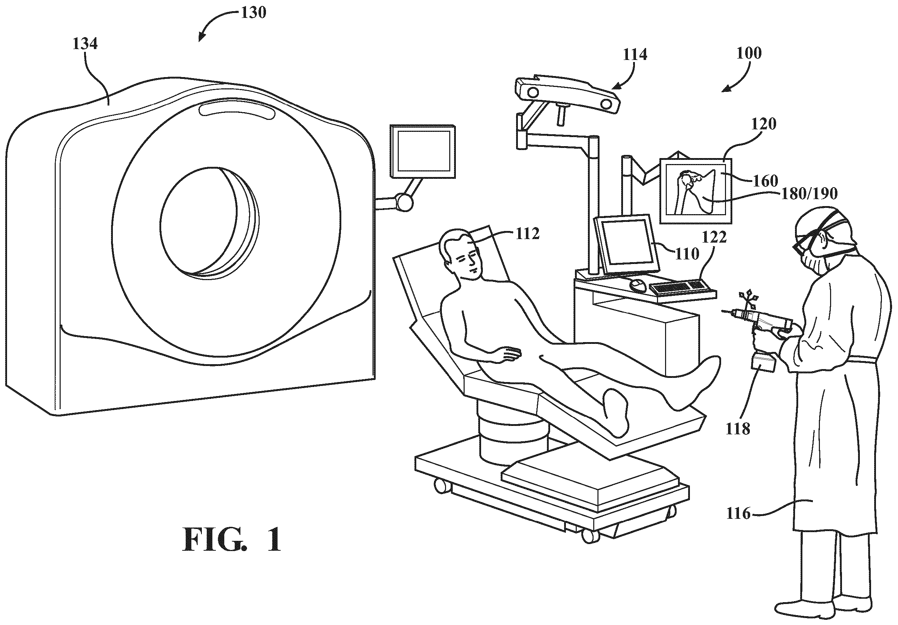

FIG. 1 is a perspective view of an operating room including a surgical planning system 100 configured to assist a user 116 in visualizing a patient shoulder anatomy and/or planning a prospective surgery on a patient 112. The surgical planning system 100 may include a controller 110, a display 120, a user input device 122, and optionally, a localizer 114. The surgical planning system 100 may be used in conjunction with a tool 118 and/or an imaging system 130. The controller 110 of the surgical planning system 100 may be in communication with the display 120 and the user input device 122. In certain implementations, the controller 110 is also in communication with the imaging system 130 and/or the localizer 114. For example, there may be a wired or wireless connection between the controller 110, the imaging system 130 and/or the localizer 114 and other components of the system. The controller 110 may facilitate communication between the various elements 130, 114, 122, 120 of the system 100. In other aspects, the controller 110 may be part of a personal computer, laptop computer, tablet computer, other suitable computing device, or the plurality of suitable computing devices, which may be collectively referred to as a computing system. The controller 110 may be implemented as one or more processors. In some implementations, the controller 110 may be integrated with the display 120 or the imaging system 130, or it may be implemented by multiple elements of the surgical planning system 100. The controller 110 may be configured to receive, send, or process data between the display 120 and the imaging system 130, or other computing devices. Further, the controller 110 may be configured to receive input from the user input device 122 to run software, send data, receive data, or manipulate the data shown on the display 120.

As described above, the surgical planning system may be in communication with localizer 114. In some implementations, the localizer 114 is an optical localizer and includes a camera unit with an outer casing that houses one or more optical sensors configured to sense movement of the various trackers. To this end, any one or more of the trackers may include active markers (not shown in detail). The active markers may include light emitting diodes (LEDs). Alternatively, the trackers may have passive markers, such as reflectors which reflect light emitted from the camera unit or another predetermined light source. In other implementations, the localizer 114 may be electromagnetically (EM) based. For example, the navigation system may include an EM transceiver coupled to the navigation controller and/or the controller 110. The localizer 114 may also be radio frequency (RF) based. In such a case, the localizer 114 may include an RF transceiver coupled to the navigation controller and/or to the controller 110. Here, the trackers may include RF emitters or transponders, which may be passive or may be actively energized. The RF transceiver transmits an RF tracking signal, and the RF emitters respond with RF signals such that tracked states are communicated to (or interpreted by) the navigation controller. The navigation controller can determine location of the RF tracker by virtue of the distance of the RF emitter or transponder relative to the RF transceiver and/or the angle (direction) of the RF emitter or transponder relative to the RF transceiver. In other implementations, the surgical planning system does not include the localizer 114.

The display 120 may be a monitor, the screen of a laptop computer, or an extended reality display device. For example, the display 120 may be a headset configured to be worn by the user 116. In some implementations, the display 120 may be configured as an extended reality device configured to execute any of the graphical functions described herein. The extended reality device may be implemented by a hand-held device (e.g., tablet or smart phone) or a head-mounted device. The extended reality device may be configured to superimpose, overlay, or combine any of the described computer-generated graphics with real-world views to implement an extended reality, augmented reality, and/or mixed reality experience for the user 116. The real-world views may be acquired directly by the eyes of the user 116 or may be a real-world video stream captured by one or more cameras of the extended reality device. When a head mounted device is utilized, the head-mounted device may include a transparent lens, or one or more display screens positioned directly in front of the eyes of the user 116 to display the computer-generated graphics relative to the real-world views.

The controller 110 may be configured to cause the display 120 to display various screens for assisting the user 116 in visualizing patient shoulder anatomy and/or planning a prospective surgery on a patient 112 as described herein. In some implementations, the controller 110 may be configured to display a graphical user interface (GUI 160) including interactable elements such as check boxes, buttons, sliders, drop-down lists, etc. on the display 120. According to some aspects, the GUI 160 may allow the user to further control the position/orientation of renderings and/or models displayed on the display 120.

The user input device 122 may be engaged by the user 116 to provide input to the controller 110 and/or manipulate objects shown on the display 120, such as interactive elements of the GUI 160, for example by clicking, dragging, scrolling, typing, or any combination of these action to provide input to the controller 110. In some implementations, the user input device 122 may be a keyboard, mouse, touch pen, track ball, a microphone, a navigated instrument, or a combination thereof. Additionally or alternatively, the user input device 122 may be implemented as a touch screen, such as integrated with the display 120, that allows the user 116 to directly interact with objects shown on the display, such as interactive elements of the GUI 160, via touch inputs/gestures through the touch screen.

In instances where the surgical planning system includes the extended reality device, the user input device may be the cameras on the extended reality device that track bodily movements, such as specific hand gestures, to particular commands, e.g., pinch to select, swipe to scroll, etc.

The imaging system 130 may include an imaging device, such as a CT machine 134, an MRI machine, an X-ray machine, or any other type of intra-operative and/or pre-operative imaging device, along with a display and computer. An exemplary X-ray machine is a C-arm. Depending on the implementation, the imaging device may generate different types of image data, such as a fluoroscopy image, an X-ray image, an MRI image, or a CT image. For example, if the imaging device is a CT machine 134, the imaging system 130 may generate CT image data. In some implementations, the imaging device may be further configured to generate 3D models of the anatomy being imaged, for example the patient shoulder anatomy. The imaging system 130 may be configured to generate one or more types of imaging data or combine types of imaging data. The imaging system 130 may transmit the image data obtained by the imaging device to the controller 110 to be processed, displayed, or otherwise manipulated by the controller 110.

The controller 110 may utilize a segmentation algorithm, such as a deep learning network or deep learning model, to generate the 3D models of patient shoulder anatomy from the image data. The controller 110 may be configured to use the algorithm to generate a two-dimensional and/or three-dimensional model including at least a portion of the patient shoulder anatomy from the image data. The controller 110 may be configured to display the 3D models generated by the algorithm on the display 120. The 3D model may further include indicators corresponding to anatomical locations, or virtual objects representing anatomical landmarks, or other modifications not specifically described herein. The indicators and/or the locations of the indicators may be determined by a deep learning model or other image processing techniques. According to an aspect, the indicators may be generated by fitting a non-patient specific model with indicators to the patient-specific 3D model. According to other aspects, segmentation algorithms that are not a deep learning network or a deep learning model may be used. The controller may optionally implement one or more smoothing operations (i.e., smoothing algorithm) to generate the 3D models). Further, the controller 110 may be configured to utilize segmentation to facilitate alert zone planning, desired tool boundaries, implant positions and/or orientation, and other features of surgical navigation and preoperative planning based on the various virtual objects described below.

Additionally, the user 116 may provide input to the controller 110 using the user input device 122 to manually or semi-automatically edit the 3D model generated by the segmentation algorithm. Exemplary manual and semi-automatic segmentation tools are described in U.S. Patent Publication No. 2019/0340765, which is hereby incorporated by reference. Alternatively, the controller 110 may receive the image data along with an output of a segmentation algorithm (the 3D models) directly from the imaging system 130.

As previously mentioned, the controller 110 may be configured to cause the display 120 to show a GUI 160, patient image data, or combination thereof. Patient image data (e.g., pre-operative patient images or intraoperative patient images), and/or renderings of 3D models of the patient shoulder anatomy may be displayed within dedicated windows of the GUI 160. The display 120 may be configured for the user 116 to interact with objects displayed as part of the GUI 160 (such as renderings of 3D models) via the user input device 122. Further, the controller 110 may be configured to cause the display 120 to show representations of anatomical landmarks of the patient shoulder anatomy, measurement values corresponding to the patient shoulder anatomy, and/or representations of planned reconstructions or interventions of the patient shoulder anatomy. In addition, the controller 110 may also be configured to simulate motion of one or more components of the patient shoulder anatomy on the display 120.

The controller 110 may be configured to cause the display 120 to show a surgical plan for a medical procedure or provide options and outcomes for various surgical planning purposes or show other features of the patient shoulder anatomy. Other features of visualizing the patient shoulder anatomy may exist, and those not specifically discussed herein may also be shown on the display 120.

In some implementations, the GUI 160 may include a three-dimensional (3-D) view window, a view options window, a patient information window, an implant family window, a workflow-specific tasks window, and/or an alignment measures window. The 3-D view window allows the user to view and interact with medical imaging data, 3-D bone models, and 3-D implant component CAD models. The view options window provides widgets to allow the user to quickly change the view of the models of the bone, models of the implant components, and alignment axes, to a desired view. The patient information window displays the patient's information such as name, identification number, gender, surgical procedure, and operating side (e.g., left humerus, right humerus). The implant family window provides drop-down menus to allow the user to select and de-select a desired implant component from a library of implant components. The workflow-specific tasks window includes various widgets to provide several functions illustratively including: guiding the user throughout different stages of the planning procedure; allowing the user to select and de-select desired alignment goals from a set of alignment goals; allowing the user to adjust the implant component(s) and bone models in desired clinical directions; displaying measured values of the alignment and position of the component(s) on the bone(s); and displaying a summary of the plan. The window may display the alignment and position information of the implant components on the bone models. For example, the window may include information related to the humerus - glenoid angle, humeral neck axis alignment parameters, and projected reconstruction parameters. Overall, the layout of the GUI 160 provides the user with a convenient roadmap and visual display to successfully plan a shoulder arthroplasty procedure, bone block placement, or lesion repair.

The GUI 160 may include one or more configurations, each of which may include objects for displaying and/or interacting with the views shown in the Figures. As mentioned above, the GUI 160 may be configured to allow the user 116 to manipulate the information shown on the display 120. For example, the user 116 may interact with the GUI 160 to view one or more renderings of the 3D models of the patient shoulder anatomy by rotating, switching views, or otherwise manipulating renderings of the 3D models. The user 116 may interact with the GUI 160 to view the patient image data from the imaging system 130. The GUI 160 may be configured to prompt the user 116 to enter information or generate surgical plans in accordance with instructions entered by the user 116. The user 116 may use the GUI 160 and/or the user input device 122 to input patient data or modify surgical plans. The patient data, in addition to the patient images, may include additional information related to the type of surgical procedures being planned, the patient's anatomical features, the patient's specific medical condition, and/or operating settings for the surgical procedures. The user 116 may mark locations of interest on the 3D models displayed by display 120 using the GUI 160. For example, the GUI 160 may include buttons to hide and/or show portions of the renderings of the 3D models, measurements such as distance between locations of interest, or other aspects of the 3D models. Utilizing the user input device 122, the user 116 may click to drag the image on the display 120 to rotate the view of the rendered 3D model, translate the view of the model, pan and/or zoom, or otherwise manipulate the information shown on the display 120 in any suitable way. The controller 110 may record the actions of the user 116 to facilitate future surgical planning, or it may record desired views, slices, or other manipulations of the renderings of the 3D model. For example, the user 116 may adjust parameters of a surgical plan for shoulder surgery (such as making modifications to an automatic and/or pre-set plan). These adjustments may be recorded by the controller 110 for future reference, and to facilitate surgical planning. Additionally, the user 116 may select a button contained within the GUI 160 to record a particular perspective of the rendered 3D model on the display 120. After further manipulation of the rendered 3D model, the user 116 may refer back to the recorded perspective to facilitate surgical planning. The user 116 may input various anatomical dimensions related to the patient shoulder anatomy, such as the size and shape of a humerus and other anatomical structures of the patient shoulder anatomy. The user input device 122 and/or the GUI 160 may also be configured to allow the user 116 to select, edit, or manipulate the patient data. For example, the user 116 may identify and/or select anatomical features from the patient data via the user input device 122 (clicking with a mouse, touch input, dragging to select, etc.).

The controller 110 may be configured to receive a set of motion data that describes the movement of a glenohumeral joint, specifically a scapula and a humerus. The motion data may be kinematic data and include translation trajectory data and/or rotation trajectory data. The motion data may include translation trajectories and/or rotation trajectories that may be used to understand how a patient's scapula and a humerus move relative to one another. The motion data may include simultaneous movement of a scapula and a humerus relative to a reference point, and further include advanced trajectory data, such as shifting centers of rotation of either of the bones, healthy motion data, or simultaneous translation and/or rotation trajectories. In addition, the motion data may include movement of the bones in multiple degrees of freedom for translation, rotation, or both.

The method may include receiving CT scan data, which may be segmented with a segmentation algorithm to generate one or more 3D models of the patient's humerus and scapula. The 3D models may be aligned with the motion data to generate kinematic 3D models of the patient's humerus and the scapula. Further, the controller 110 may be configured to generate a humerus 3D model 180 and a scapula 3D model 190 and cause the display 120 to show the humerus and scapula 3D models 180,190 with the motion data applied to them, thereby simulating the motion of a glenohumeral joint on the display 120 for the specific patient.

The controller 110 may further generate and/or cause the display 120 to show one or more virtual objects relative to the representations of patient shoulder anatomy. The virtual objects (which may be referred to as "objects" in this description) may be based on the image data of patient shoulder anatomy and may include an area, a volume, a point, a line, a plane, or any other shape not disclosed herein to represent anatomical aspects of patient shoulder anatomy (such as a glenoid, a glenoid projection virtual object, a glenoid track virtual object, a glenoid track line virtual object, an insertion virtual object, etc.). Additionally, the controller 110 may generate portions of the humerus 3D model 180, including a humeral head portion 182, and the scapula 3D model 190 that may correspond to real portions of the patient shoulder anatomy upon which the 3D models 180, 190 are fitted.

### II. Segmentation Algorithm & Identification of Lesion

One exemplary way of automated segmentation is described in U.S. Patent No. 8,971,606, entitled, "Method for automatically identifying the contours of a predefined bone, derived methods and corresponding computer program products", the disclosure of which is hereby incorporated by reference. There may be various other ways in which to perform automated segmentation, and the techniques are not limited to automated segmentation using techniques described in U.S. Patent No. 8,971,606. As one example, segmentation of the CT image data to yield segmented objects includes comparisons of voxel intensity in the image data to determine bony anatomy and comparisons to estimated sizes of bony anatomy to determine a segmented object. Moreover, as described above, the example techniques may be performed with non-automated segmentation techniques, where a medical professional evaluates the image data to segment anatomical objects, or some combination of automation and user input for segmenting anatomical objects. A computing device, such as the controller 110, may generate segmented image data of the patient anatomy in order to create anatomical objects. It should be appreciated that the controller 110 may receive the segmented image data from other computing devices, unchanged as the imaging system 130.

In one or more examples, the controller 110 may utilize image data to compare the patient anatomy against a fitted statistical shape model (SSM) as a way to determine characteristics of the patient anatomy prior to the patient suffering the injury or disease (e.g., compare the size, shape, orientation, etc.). In some examples, the controller 110 may compare 3D point data of non-pathological points of anatomical objects of patient anatomy in the image data to points in the SSM. In other words, the method may include determining a surface model of the patient shoulder anatomy from the patient image data and fitting the determined surface model of the patient shoulder anatomy to a statistical shape model. The contours of this instantiated shape model may be representative of the patient anatomy prior to the patient suffering injury or disease.

Other methods of segmentation are described in International Patent Publication No. 2020205248, entitled "Pre-morbid characterization of anatomical object using statistical shape modeling (ssm)" and/or U.S. Patent Application Ser. No. 17/607323, entitled "Automated planning of shoulder stability enhancement surgeries", the disclosures of both of which are hereby incorporated by reference in their entirety.

### III. Motion Data

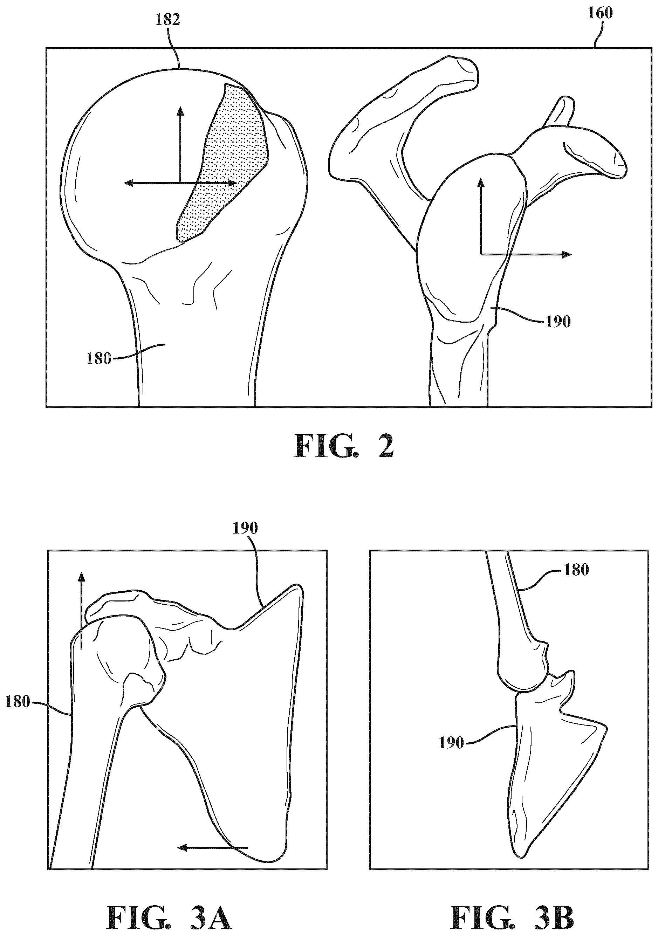

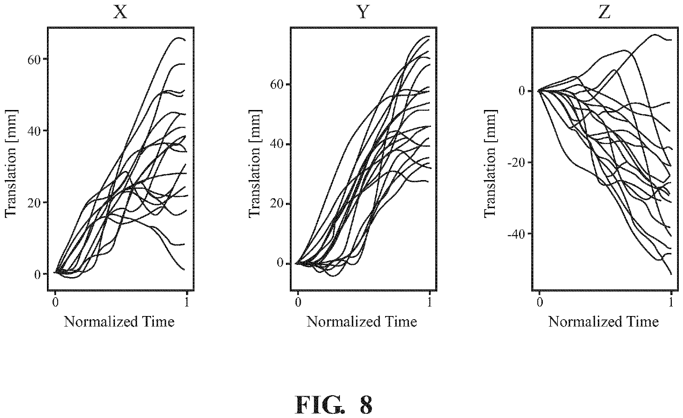

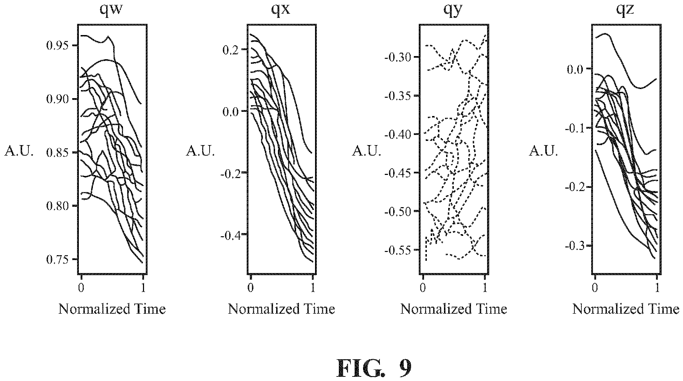

Referring to FIGS. 3A-7B, the controller 110 may be configured to receive motion data of one or more kinematic motions of a glenohumeral joint, each one including translation trajectories and rotation trajectories, for non-patient persons. Each of the kinematic motions includes translation trajectories and rotation trajectories for the humerus and the scapula of a population of non-patient persons. As shown in FIG. 8, the translation trajectories may be represented as a magnitude in each of the x, y, and z directions. The peak of each graph represents the maximum translation in that direction with respect to time data. As shown in FIG. 9, the rotation trajectories are represented in similar graphs as computed rotation averages calculated by quaternion averaging. Similarly, each graph demonstrates the maxima of rotation for that kinematic motion.

The motion data includes measured values that may be obtained from measurements of a population of non-patient persons, including both uninjured and injured populations. The translation and rotation trajectories may be measured relative to any part of the anatomy. For example, translation trajectories may describe the motion of a scapula relative to a humerus, or the motion of the humerus relative to the scapula. Similarly, the rotation trajectories may utilize any point of the anatomy as an origin point for rotation. The motion data may represent a variety of joint motions, and this disclosure is not limited to the anatomy specifically disclosed herein, and could apply to other joints beyond the shoulder, such as rotation for the femur relative to the pelvis.

### IV. Relative Pose

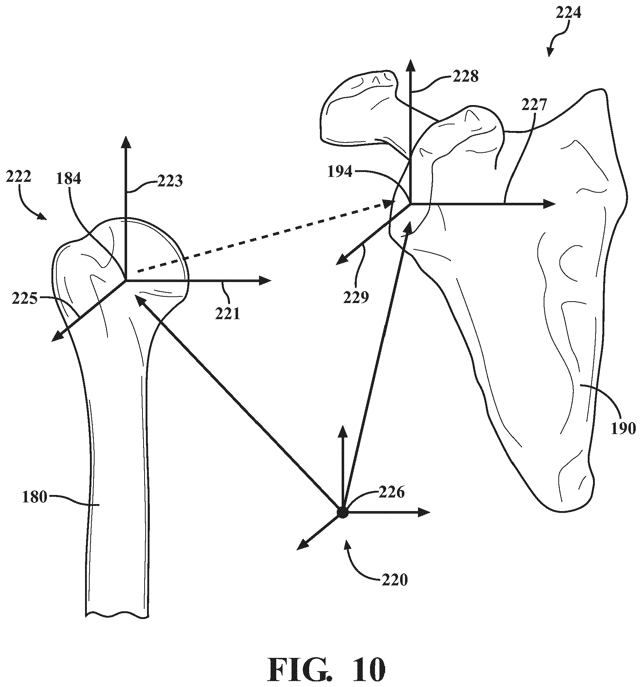

After receiving the motion data, the controller 110 may be configured to identify the relative movement between a humerus and a scapula, which may be simulated by the movement between the humerus 3D model 180 and the scapula 3D model 190. Referring to FIG. 10, the motion data may have a biplane fluoroscopy format, which represents translation trajectory data and rotation trajectory data in a biplane coordinate system 220. Additionally, or alternatively, the humerus 3D model 180 and the scapula 3D model 190 may include independently defined local coordinate systems based on certain anatomical features as described below.

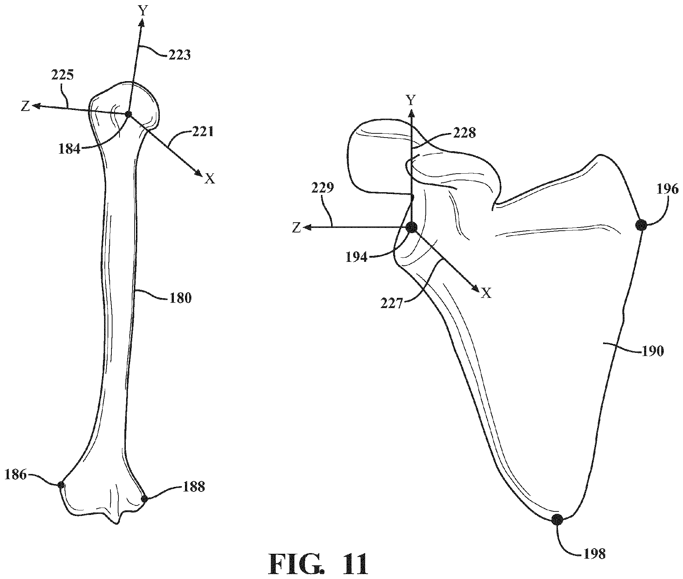

Referring to FIG. 11, the controller 110 may be configured to generate representations of anatomical features on the humerus 3D model 180 based on anatomical features of a patient's humerus including one or more of a humeral head center point 184, a lateral epicondyle object 186, a medial epicondyle object 188, a humeral shaft axis, and a greater tuberosity. There are many other anatomical features on a humerus that may be of interest, and those not specifically disclosed herein may be generated/utilized in addition to those disclosed. The controller 110 may be further configured to generate representations of patient's anatomical features on the scapula 3D model 190 including one or more of a glenoid center point 194, an inferior angle 198, a trigonum spinae object 196, a posterior lateral acromion, an acromioclavicular joint, a scapular spine axis, and a scapular wing plane. There are many other anatomical features on a scapula that may be of interest, and those not specifically disclosed herein may be utilized in addition to those disclosed. The controller 110 may apply an algorithm or run a program to generate representations of these anatomical features or allow the user 116 to manually identify these anatomical features on the humerus and/or scapula 3D models 180, 190. For example, a user 116 may use the user input device 122 and the GUI 160 to select anatomical features on the display 120 and record the locations of those anatomical features.

The controller 110 may be configured to generate a local humerus coordinate system 222 by generating planes and/or similarly representative objects (e.g., lines) between the anatomical features described above. For example, the origin of the local humerus coordinate system 222 may be defined as the humeral head center point 184. The controller 110 may be configured to create a first line connecting the lateral epicondyle object 186 and the medial epicondyle object 188 and identify a midpoint of the first line. The controller 110 may be configured to create a second line connecting the midpoint of the first line to the humeral head center point 184, which may point in the same direction as the Y-axis 223 of the local humerus coordinate system 222. The controller 110 may be configured to create a first plane containing the humeral head center point 184, the lateral epicondyle object 186, and the medial epicondyle object 188, and a second plane normal to the first plane and containing the humeral head center point 184 and the midpoint of the first line. The second plane may be aligned with and/or include the X-axis 221 of the local humerus coordinate system 222. The controller 110 may be configured to create a third plane based on a determined Z-axis 225. The Z-axis is determined based on the cross product of the X-axis and the Y-axis of the local humerus coordinate system 222. The third plane is orthogonal to the first and second planes and includes the Z-axis 225.

The local humerus coordinate system 222 may define the orientation and/or a pose of the humerus 3D model 180 relative to the origin, which is the humeral head center point 184 in this embodiment. It is to be understood that other configurations of the local humerus coordinate system 222 exist, and it may be defined utilizing different anatomical features and/or alternate relationships than those specifically described herein. Additionally, the controller 110 may be configured to cause the display 120 to show the local humerus coordinate system 222 relative to the humerus 3D model 180. For example, referring to FIG. 11, the controller 110 may cause the display 120 to show the X-axis 221, Y-axis 223, and Z-axis 225 of the local humerus coordinate system 222. Additionally or alternatively, the controller 110 may generate virtual objects to represent the relationships between the anatomical features described above, including planes, lines, or any other shape to be shown on display 120.

The controller 110 may be configured to generate a local scapula coordinate system 224 by generating planes and/or similarly representative objects (e.g. lines) between the anatomical features. For example, the origin of the local scapula coordinate system 224 may be defined as the glenoid center point 194. The controller 110 may be configured to create a line connecting the glenoid center point 194 and the trigonum spinae object 196 which may be aligned with the Z-axis 229 of the local scapula coordinate system 224. The controller 110 may be configured to create a plane including the glenoid center point 194, the trigonum spinae object 196, and the inferior angle 198. A line perpendicular to the plane including the glenoid center point 194, the trigonum spinae object 196, and the inferior angle 198 may be created with a second plane perpendicular to the first plane and including the trigonum spinae object 196 and the glenoid center point 194. This perpendicular line may be aligned with the X-axis 227 of the local scapula coordinate system 224. The controller 110 may be configured to generate the Y-axis 228 of the local scapula coordinate system 224 based on the cross product of the X-axis 227 and Z-axis 229 of the local scapula coordinate system 224. The controller 110 may be configured to generate a third plane perpendicular to the first and second planes, which includes the Y-axis 228.

The local scapula coordinate system 224 may define the orientation and/or a pose of the scapula 3D model 190 relative to the origin, which is the glenoid center point 194 in this embodiment. It is to be understood that other configurations of the local scapula coordinate system exist, and it may be defined utilizing different anatomical features and/or alternate relationships than those specifically described herein. Additionally, the controller 110 may be configured to cause the display 120 to show the local scapula coordinate system 224 relative to the scapula 3D model 190. The controller 110 may generate virtual objects to represent the relationships between the anatomical features described above, including planes, lines, or any other shape to be shown on display 120.

To accurately apply the non-patient motion data to the patient's humerus 3D model 180 and the scapula 3D model 190, the controller 110 may be configured to align the biplane coordinate system 220 of the motion data with the local humerus coordinate system 222 and the local scapula coordinate system 224. The biplane coordinate system 220 may also be referred to as a global coordinate system and/or a common reference frame. As shown in FIG. 10, after generating the local coordinate systems 222 and 224, the humerus and scapula 3D models 180 and 190 may be aligned by translating each respective local coordinate system to a global origin 226. The global origin 226 may be determined automatically by the controller 110, or the controller 110 may be configured to allow the user 116 to manually select the global origin 226 (e.g., with the user input device 122). After translation of the local coordinate systems, an angle difference may be measured in all three directions (x, y, and z) based on the difference between the biplane coordinate system 220 and the local humerus and local scapula coordinate systems 222 and 224. The controller 110 may be configured to rotate the corresponding 3D models by the angle differences in each direction to align the local coordinate systems to the biplane coordinate system 220. Still referring to FIG. 10, the local humerus and local scapula coordinate systems 222, 224 are adjusted in accordance with the angle differences, aligning the two systems to the same reference coordinate system 220. In other implementations, one of the local coordinate systems 222, 224 may be treated as the global coordinate system. For example, the local humerus coordinate system 222 may be used as a reference coordinate system, and the biplane coordinate system 220 and the local scapula coordinate system 224 may be aligned to the reference coordinate system as described above.

### V. Applying Motion Data

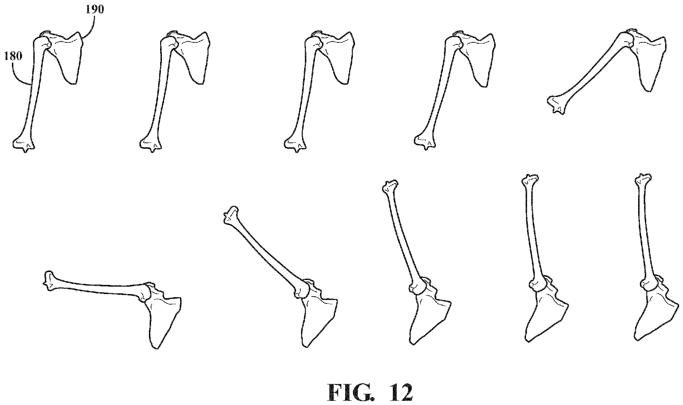

The controller 110 may be configured to run a software and/or algorithm to apply the motion data to the humerus 3D model 180 and the scapula 3D model 190 of the patient and display renderings of the humerus 3D model 180 and the scapula 3D model 190 with the applied motion data on the display 120 (as described below). The controller 110 may cause the display 120 to show the starting and ending poses of each kinematic motion and/or the movement of the 3D bone models 180, 190 in between those poses. The controller 110 may be configured to pause the motion at any point before, during, or after the kinematic motion, and record the position of the humerus 3D model 180 relative to the scapula 3D model 190 and/or the position of the scapula 3D model 190 relative to the humerus 3D model 180. The controller 110 may be configured to cause the display 120 to show the motion data in many different configurations, such as the configurations shown in the Figures and described above. The controller 110 may be configured to cause the display 120 to show the motion data through a video in which the motion is continuous. For example, as shown in FIG. 3A, a first pose of the humerus 3D model 180 and the scapula 3D model 190 may be shown, then continuous movement may be shown on the display 120 until the humerus 3D model 180 and the scapula 3D model 190 reach a second pose shown in FIG. 3B. Alternatively, as shown in FIG. 12, the controller 110 may be configured to cause the display 120 to show any number of time stamps of the continuous movement and display a series of poses of the humerus 3D model 180 and the scapula 3D model 190. These time stamps may be equidistant (e.g., at the same time increments between the initial and terminal poses), or at other times during the kinematic motion.

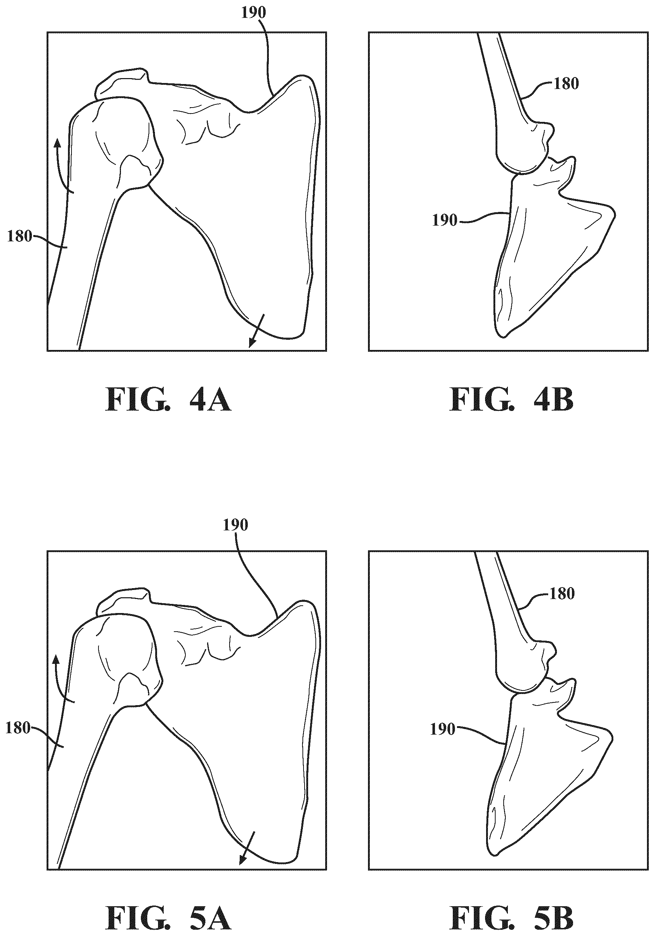

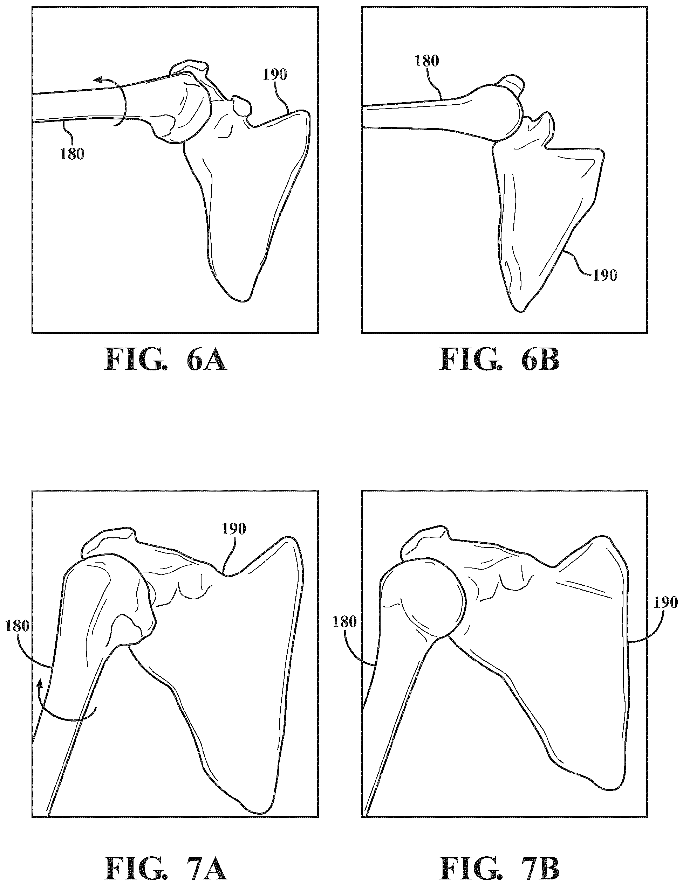

The kinematic motions of the glenohumeral joint included in the motion data may include one or more of forward elevation, scapula abduction, coronal abduction, external rotation at 90° of abduction, and external rotation at rest. Examples of these kinematic motions applied to the humerus 3D model 180 and the scapula 3D model 190 are shown in FIGS. 3A - 7B. A first and terminal pose of forward elevation are shown in FIGS. 3A and 3B, respectively. Similarly, a first and terminal pose of scapula abduction are shown in FIGS. 4A and 4B. FIGS. 5A and 5B respectively show a first and terminal pose of coronal abduction. Coronal abduction is also represented in FIG. 12 as a series of 10 images with 9 equidistant time intervals between them. A first and terminal pose of external rotation at 90° of abduction are shown in FIGS. 6A and 6B, and a first and terminal pose of external rotation at rest are shown in FIGS. 7A and 7B. It is to be understood that there are many kinematic motions relative to the glenohumeral joint, and those not specifically disclosed herein may be represented as motion data and received by the controller 110 and utilized to complete an assessment of the glenohumeral joint.

The controller 110 may be further configured to record the motion of the renderings of the 3D bone models 180, 190 on the display 120 after applying the motion data. For example, the positions of the scapula 3D model 190 relative to the humerus 3D model 180 may be recorded by the controller 110 to facilitate future surgical planning and/or be shown on the display 120 at a later time. The positions of the scapula 3D model 190 relative to the humerus 3D model 180 may be represented by a virtual object indicating the area of contact between the two bones, or by any other shape or series of shapes which indicates the interaction between the scapula 3D model 190 and the humerus 3D model 180.

### VI. Terminal Position

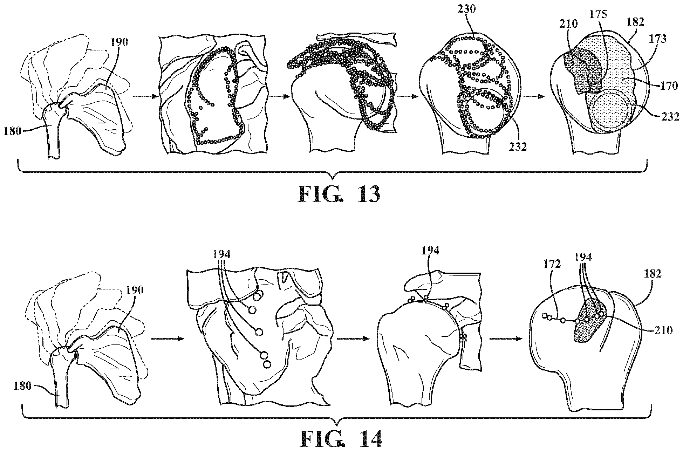

After applying the motion data to the humerus 3D model 180 and the scapula 3D model 190, the controller 110 may be configured to determine a position of the scapula 3D model 190 relative to the humerus 3D model 180 to assess the state of the glenohumeral joint. In some implementations, the controller 110 may be configured to generate positions of the scapula 3D model 190 and the humerus 3D model 180, as shown in FIG. 13, and represent those positions on the display 120 with a glenoid outline 230 approximating the shape of the glenoid on the humerus 3D model 190 for all of the various relative positions of the humerus 3D model 180 and scapula 3D model as simulated in the motion data. In the second from left image, the outline of the outer bounds of the engagement of the humerus with the glenoid is projected on the scapula. In the middle image of FIG. 13, the projections of all the outlines of various engagement points of the humerus and scapula are collectively displayed relative to the humerus and the scapula across the various positions of the scapula and humerus assumed in the motion data. In the second image from right, the projections of all of the outlines of the various engagements of the humerus and scapula across the various positions of the scapula and humerus assumed in the motion data and collectively displayed relative to just the humerus (referred to as the glenoid outline 230), including an outline of engagement of a terminal position 232 of the humerus with the scapula.

The glenoid outline 230 may be formed with a series of points on the humerus 3D model 190 and may be obtained from a statistical shape model to accurately approximate the anatomical characteristics of a glenoid. The glenoid outline 230 may also be depicted with a continuous line. The glenoid outline 230 represents an aggregate boundary of all of the different outlines of the engagement of the glenoid with the humerus on the humerus 3D model when the motion data is applied thereto.

In other implementations, with reference to the images of FIG. 14, the controller 110 may represent the positions of the scapula 3D model 190 and the humerus 3D model 180 with a series of glenoid center points 194 across the various positions of the scapula 3D model 190 and the humerus 3D model 180 when the motion data is applied thereto. With reference to the second image from the left of FIG. 14, the centers of the glenoid engagement (glenoid center 194) with the humerus are projected on the scapula for each of the plurality of relative positions of the humerus and scapula simulated in the motion data. In the image second from the right, the projections of all the center points for the various engagement of the humerus and scapula are collectively displayed relative to the humerus and the scapula for each relative position of the humerus and scapula simulated by the motion data. In the rightmost image, the centers of all of the projections of the various engagements of the humerus and scapula and collectively displayed relative to just the humerus. Each center point displayed on the rightmost image represents the center of the glenoid with respect to the humerus, and the series of glenoid of center points represents the various locations of the center of the glenoid as projected onto the humerus 3D model based on the various relative positions simulated by the motion data.

In some implementations, the controller 110 may be configured to determine any number of positions of the humerus 3D model 180 relative to the scapula 3D model 190 and project the representations of the glenoid outline 230 and/or the glenoid center points 194 onto the humerus 3D model 180, as shown in FIGS. 13 and 14.

Referring again to FIG. 13, the one or more positions of the humerus 3D model 180 relative to the scapula 3D model 190 that are generated by the controller 110 may be used to visualize a glenoid track virtual object 170, which may be used to evaluate the state of the joint and/or the impact of a lesion, such as lesion virtual object 210, on the interaction between the humerus 3D model 180 and the scapula 3D model 190. The glenoid track virtual object 170 may be represented by a variety of shapes generated by the controller 110, such a plane, a set of lines, or a series of circles. Furthermore, the glenoid track virtual object 170 may feature a number of characteristics including a glenoid track length, a glenoid track distance, a glenoid track area, a glenoid track angle, and combinations thereof. Each of these characteristics may be communicated to the user in text or graphics via the display. In the illustrated configuration, glenoid track virtual object 170 defines an area representative of all the area on the humerus that contacts the glenoid across the various positions of the scapula and humerus simulated by the motion data. The glenoid track virtual object 170 may include a medial border 173 and a lateral border 175 to delineate the glenoid track virtual object 170 from the rest of the humeral head portion 182. The lateral border 175 of the glenoid track virtual object 170 may be based on one or more anatomical landmarks.

Referring again to FIG. 14, the controller 110 may be configured to utilize the one or more positions of the humerus 3D model 180 relative to the scapula 3D model 190 to visualize a glenoid track line 172. The glenoid track line 172 would include two or more glenoid center points 194 representing the positions of the humerus 3D model 180 relative to the scapula 3D model 190 generated by the controller 110. Similarly to the glenoid track virtual object 170, the glenoid track line 172 may be used to assess the state of a glenohumeral joint and/or the impact of a lesion on the interaction between a humerus and a glenoid. It is to be understood that any combination of positions of a humerus relative to a scapula may be utilized for this purpose and any configuration of the glenoid center points 194 and/or representations of the glenoid outline 230 may be shown on the display 120, and those not specifically disclosed herein may be utilized. For example, the controller 110 may control an indicator, such as a widget on the GUI, to indicate that the lesion virtual object 210 has a certain track engagement, such as on-track or off-track, based on the position of the lesion virtual object 210 and the glenoid track line 172, such as indicating that the lesion virtual object 210 is on track if the lesion virtual object 210 intersects the glenoid track line 172.

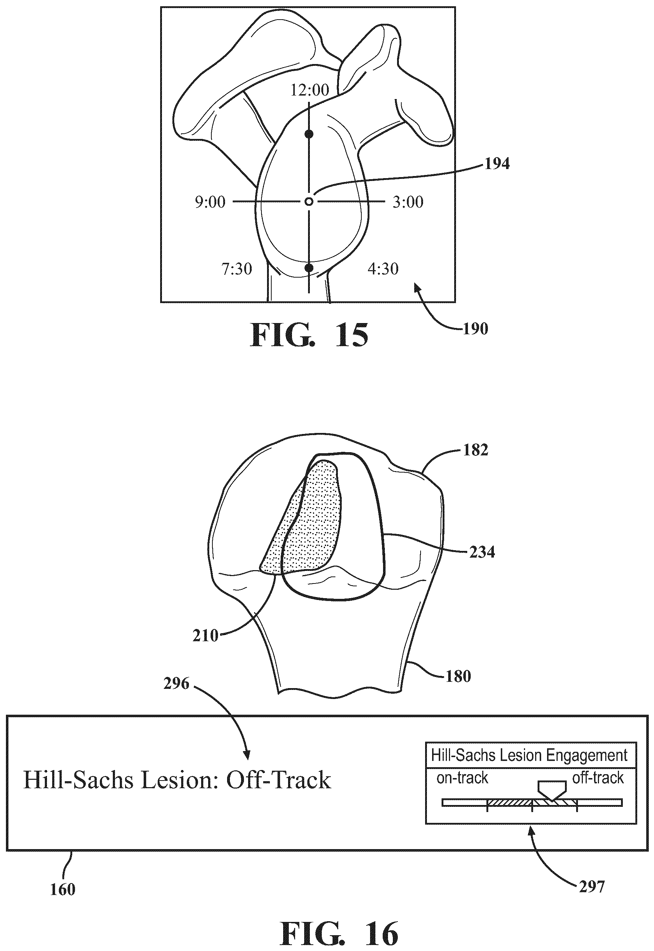

In other implementations, the controller 110 may be configured to determine and/or record only certain positions of the humerus 3D model 180 relative to the scapula 3D model 190. For example, the controller 110 may be configured to determine a terminal position 232 of the humerus 3D model 180 relative to the scapula 3D model 190. As shown in FIG. 13, the terminal position 232 may be the last position in accordance with the motion data applied to the humerus 3D model 180 and the scapula 3D model 190. Additionally, the terminal position 232 may represent the maximum position and/or the maximum rotation of the humerus 3D model 180 relative to the scapula 3D model 190, or both. Of course, the motion data includes a number of alternate positions other than the terminal position that represent something other than the maximum position and/or the maximum rotation of the humerus 3D model 180 relative to the scapula 3D model 190. Accordingly, the controller 110 may be configured to determine the terminal position 232 for each of the kinematic motions included in the motion data that is applied to the humerus and scapula 3D models 180, 190. The controller 110 may be configured to cause the display 120 to show each of those terminal positions 232 and/or record the terminal positions 232 to facilitate surgical planning. The engagement between the glenoid and humerus at the terminal position 232 may be projected onto the humeral head portion 182 and shown on the display 120 relative to the humerus 3D model 180. With reference to FIG. 16, after the engagement of the humerus and glenoid is projected onto the humeral head portion 182 at the terminal position 232, the resultant virtual object may be referred to as a glenoid projection virtual object 234.

Referring to FIG. 15, the controller 110 may be configured to determine the location of the glenoid center points 194 using the 3, 6, 9 and 12 o'clock locations on the scapula 3D model 190. The glenoid center point 194 may be identified using the midpoint of the lines connecting the 3 and 9 o'clock locations and 6 and 12 o'clock locations. Further, the controller 110 may be configured to allow the user 116 to manually determine the glenoid center point 194 (e.g., with the user input device 122).

### VII. Track engagement

As described above with reference to FIG. 13, to assess the state of the patient shoulder anatomy, such as a glenohumeral joint, the controller 110 may be configured to generate the glenoid track virtual object 170 using the methods described above, through applied motion data. The glenoid track virtual object 170 represents the interaction between the glenoid of the scapula 3D model 190 and the humerus 3D model 180 and therefore is indicative of the contact between the two bones, which may provide an indication regarding the health of a glenohumeral joint. The controller 110 may be configured to cause the display to show the humerus 3D model 180 and the glenoid track virtual object 170. Additionally, the controller 110 may be configured to generate a virtual representation of a lesion and cause the display 120 to show a lesion virtual object 210 relative to the humerus 3D model 180. If controller 110 determines that the boundary of the glenoid track virtual object 170 overlaps with the boundary of the lesion virtual object 210 on the humeral head portion 182, the controller 110 or the user 116 may make an assessment that the lesion is an off-track lesion, which may necessitate shoulder surgery. In another configuration, if the controller 110 determines that the lesion virtual object 210 is entirely contained within the area of the glenoid track virtual object 170, the controller 110 display an indicator that the lesion is an on-track lesion, which may not necessitate shoulder surgery. A user could make a similar assessment by comparing the bounds of the glenoid track virtual object 170 with the relative bounds of the lesion virtual object 210. It is worth noting that, throughout this application, an on-track lesion is one where the Hill-Sachs lesion is contained within the glenoid track (the area of engagement between the humeral head and the glenoid). These on-track lesions are less likely to cause instability. An off-track lesion is one where the Hill-Sachs lesion extends beyond the glenoid track. These off-track lesions are more likely to engage and cause recurrent dislocation.

More specifically, the surgical planning system 100 may include a method for identifying the presence and/or location of the lesion on the humeral head portion 182, which may be known as a Hill-Sachs lesion. In some implementations, the surgical planning system 100 may include a method for identifying the presence and/or location of a lesion on the glenoid, which may be known as a Bankart lesion. In the exemplary configuration described below, the surgical planning system 100 is configured to identify the presence and/or location of a Hill-Sachs lesion, and generate and/or render virtual objects related to that lesion.

The controller 110 may be configured to apply a segmentation algorithm to patient image data to generate the humerus and scapula 3D models 180, 190 and generate the lesion virtual object 210 based on the presence and/or location of a lesion. The segmentation algorithm may be used to segment image data of the patient shoulder anatomy in order to detect the presence and/or location of a lesion. The lesion may be a Hill Sachs lesion on the surface of the humeral head portion 182.

In some implementations, the controller 110 may be further configured to run a deep learning algorithm, such as a deep learning neural network, to segment the image data of the patient shoulder anatomy, and/or generate the lesion virtual object 210 on a humerus prior to, during, or after segmentation.. The deep learning algorithm may also be configured to predict the surface area of the lesion virtual object 210 and/or the border of the lesion virtual object 210. The detection of the lesion may be initially based on the image data of the relevant patient shoulder anatomy. In some implementations, the deep learning algorithm may provide an output of the region in the form of a binary mask that includes the lesion virtual object 210 in the voxel space. The mask may then be mapped onto a 3D surface model of the segmented humeral head portion 182 to provide a rendering of the lesion virtual object 210 overlaid on the humerus 3D model 180. The deep learning algorithm may be trained with data from a population of persons and manually identified lesions. This training may allow the algorithm to accurately identify lesion virtual objects 210 on the humeral head portion 182. According to some aspects, one or more different deep learning algorithms may be used to perform various tasks, including identifying the lesion virtual object, predicting the surface area of the lesion virtual object, and predicting the border of the lesion virtual object, etc.

In some cases, the segmentation algorithm, the deep learning model, and/or the alternative methods may provide a flawed representation of a lesion. Although the approximate location and shape of the lesion virtual object 210 may be generated accurately, it is possible that the boundaries of the lesion virtual object 210 on the humeral head portion 182 remain undefined. The controller 110 may be further configured to apply one or more of the following algorithms to the humerus 3D model 180 and/or the scapula 3D model 190: edge detection, watershed analysis, curvature analysis, and/or statistical shape modeling to further refine the boundaries of the lesion virtual object 210. The controller 110 may be configured to apply one of the above algorithms to the 3D models and/or the patient image data. These algorithms may be referred to as post-processing algorithms and may include some algorithms not specifically described herein. Any one of the post-processing algorithms may be applied to the image data of the patient shoulder anatomy to generate the 3D models through segmentation or may be applied to a segmented 3D model. The output of any one of the post-processing algorithms may be the 3D model including anatomical data, where the anatomical data includes the presence and/or location of a lesion virtual object 210 on the patient shoulder anatomy. The controller 110 may be configured to cause the display 120 to show the lesion virtual object 210 relative to the humerus 3D model 180. After the application of the post-processing algorithms, the boundaries of the lesion virtual object 210 may be more accurately defined. In addition, the controller 110 may be further configured to receive input from the user input device 122 from the user 116 to manipulate the boundaries of the lesion virtual object 210 on the surface of the humeral head portion 182 to correct any flaws in the 3D model prior to post-processing.

With reference to Fig. 13, as described above, the controller's determination of track engagement may include a comparison between the glenoid track virtual object 170 and the lesion virtual object 210, such as perform an analysis based on an area of the glenoid projection virtual object 234 representative of the terminal position and a second area of the lesion virtual object 210. Comparison of the glenoid track virtual object 170 and the lesion virtual object 210 may yield one or more of the following qualitative assessments of track engagement: on-track, off track, or near track. The qualitative assessment may be based on the proximity of the lesion virtual object 210 to the glenoid track virtual object 170, the position of the lesion virtual object 210 relative to the glenoid track virtual object 170, and/or the intersection between the lesion virtual object 210 and the glenoid track virtual object 170. For example, if there is only partial overlap between the lesion virtual object 210 and the glenoid track object, the system displays an indication that the lesion is off-track. Similarly, if there is complete overlap between the glenoid track virtual object 170 and the lesion virtual object 210, the system may display an indication that the lesion is on-track. With reference to Fig. 14, it is also contemplated that the qualitative assessment may be based on the proximity of the lesion virtual object 210 to the glenoid track line 172, the position of the lesion virtual object 210 relative to the glenoid track line 172, and/or the intersection between the lesion virtual object 210 and the glenoid track line 172, alone or in combination with the assessment based on the glenoid track virtual object 170.

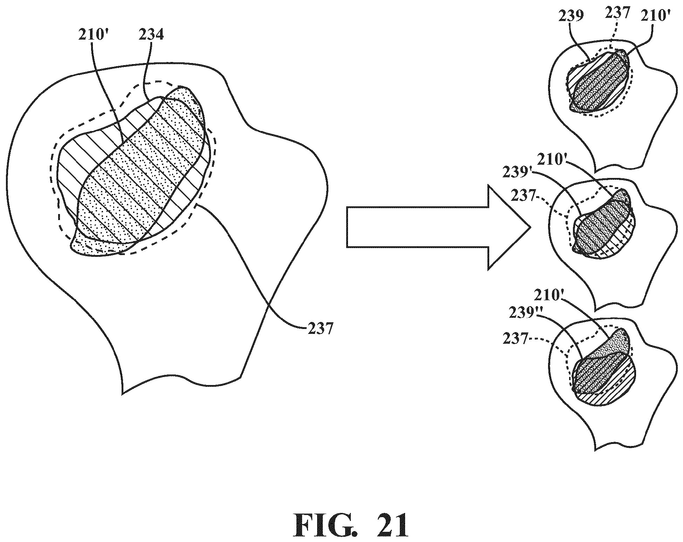

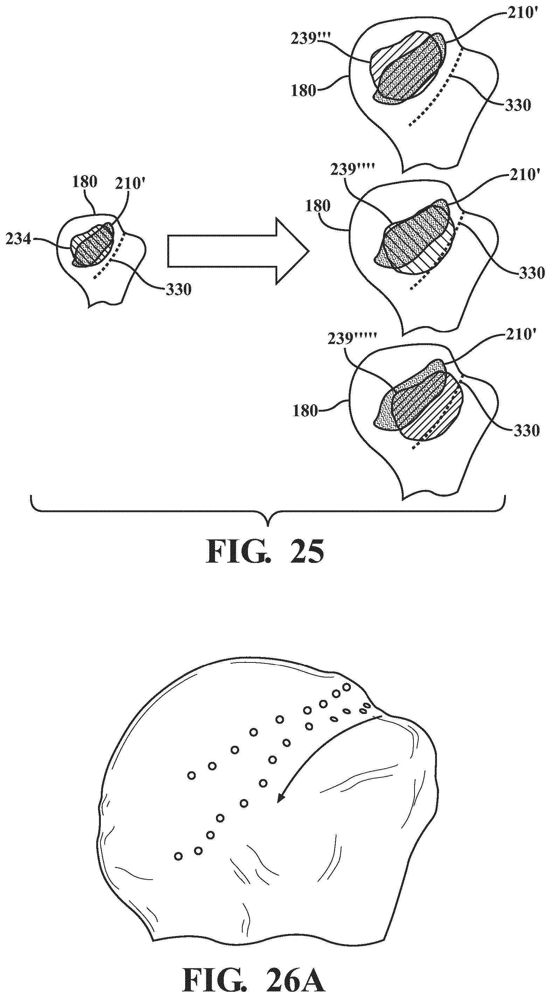

In addition, as will be described below, the controller may provide the track engagement assessment based on other information, such as based on the first area of the glenoid projection virtual object 234 and a surface of a humeral head portion of the humerus 3D model 180 (See FIG. 21), based on the area of the glenoid projection virtual object 234 and a soft tissue insertion line 330 (see FIG. 25), or based on some combination thereof. In addition, the controller 110 may perform the track engagement assessment based on other virtual objects representing anatomical features, and the track engagement is not limited to utilizing the comparisons disclosed herein. Furthermore, while track engagement refers specifically to the engagement of the bones of the glenohumeral joint, similar methods can be used for other joints in the body, such as the hip joint. In these instances, track engagement should be more broadly understood as joint engagement.

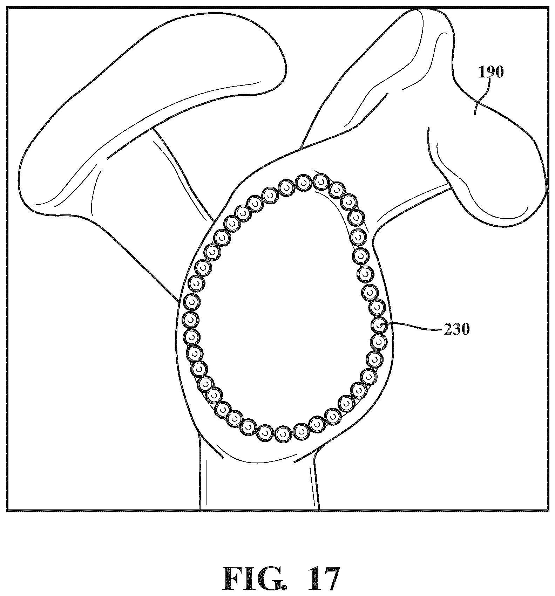

With reference to FIG. 16, the controller 110 may cause the display 120 to display a rendering of the glenoid projection virtual object 234 on the humerus 3D model 180 as an outline approximating the shape of a glenoid. Alternatively, as shown in Figure 17, the controller 110 may be configured to cause the display 120 to display a rendering of a glenoid outline 230 on a rendering of a scapula 3D model 190, according to one implementation. The glenoid projection virtual object 234 may be displayed in various ways, such as with a various line configurations, such as dash lines, or as an overlay with different opacity, with different colorization, or with different patterns.

FIG. 16 also illustrates an example of how the system may convey the indicator relating to track engagement via the display 120 which may be referred to as a first signal 296 that the lesion virtual object 210 is classified as off-track. The first signal 296 may be configured to indicate other states of the lesion virtual object 210, such as an on-track or near-track lesion. Yet another example of a track engagement indicator is an impact rating 297, which provides a degree of on-track or off-track regarding the state of a lesion. The impact rating 297 may be shown as a sliding scale. In some implementations, the impact rating 297 may be a numerical scale to indicate severity. Alternative ways to convey the impact rating may are contemplated, such as displaying certain text, a certain image, or other signal to provide a degree of on-track or off-track engagement.

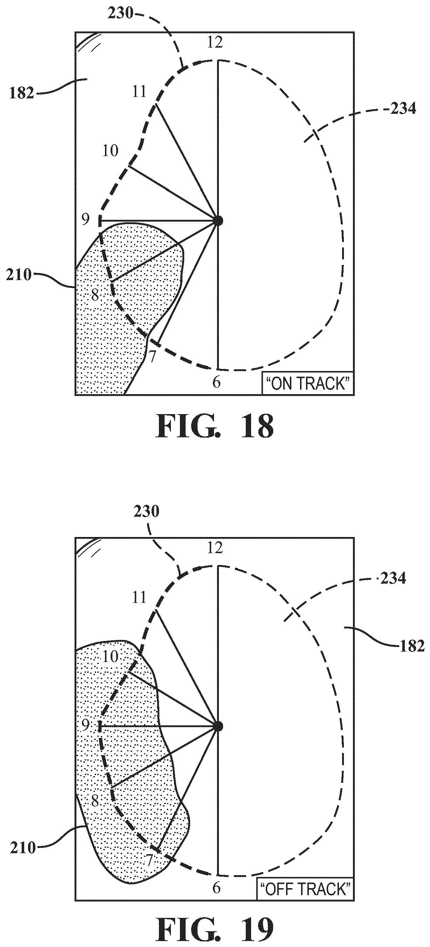

As shown in FIGS. 18 and 19, the glenoid projection virtual object 234 may be used to complete an assessment of track engagement in a different way using clockface positions relative to the humerus. In this implementation, the controller 110 may be configured to cause the display 120 to show the glenoid projection virtual object 234 relative to the humeral head portion 182 of the humerus 3D model 180. In FIGS. 18 and 19, a clockface system of the glenoid is superimposed on the humeral head portion 182 with the center of the clockface positioned at the inertial center of the glenoid projection virtual object 234. The 12-6 line is the based on the long axis of the glenoid projection virtual object 234. The 3-9 line is perpendicular to 12-6 line. The various clockface lines (show as numbers 6-12) are spaced 30 degrees apart which results in the glenoid projection virtual object 234 being divided into a plurality of separate areas. Theis clockface system is displayed on the humerus 3D model 180 along with the lesion virtual object 210 relative to the surface of the humeral head portion 182.

Referring to FIG. 18, the lesion virtual object 210 is shown as overlapping two of the areas clockface system of the glenoid projection virtual object 234. The controller 110 may assess the state of a glenohumeral joint by analyzing the location of the lesion virtual object 210 relative to the different divided areas of glenoid projection virtual object 234. For example, if the lesion virtual object 210 overlaps two or less of the areas that the clockface system of the glenoid projection is divided into, there may be little risk of interference of the lesion virtual object 210 on a glenohumeral joint.

That said, if the lesion virtual object 210 overlaps more than two of the areas of the glenoid projection virtual object 234, as shown in FIG. 19, there may be higher risk of the lesion virtual object 210 impacting a glenohumeral joint. The controller 110 may be configured to complete this assessment automatically or visualize the structures on the display 120 for the user 116 to manually determine the presence and/or degree of overlap between the lesion virtual object 210 and the glenoid projection virtual object 234. In other words, the controller 110 may be configured to provide an on-track indicator on the display screen for FIG. 18 because the controller determined that the lesion virtual object 210 overlaps two or fewer areas of the clockface system of the glenoid virtual projection 234. On the other hand, the controller 110 may be configured to provide an off-track indicator on the display screen for FIG. 19 because the controller determined that the lesion virtual object 210 overlaps more than two of the clockface system of the glenoid projection virtual object 234. Of course, it is contemplated that more areas (more than 12) or fewer areas (less than 12) may alternatively be used. The controller 110 may be configured to count the areas of overlap and provide an indicator regarding track engagement based on the number of areas of overlap.

Alternatively, the indicator for track engagement, such as whether the display screen presents on-track or off-track, may be based on amount of overlap between the lesion virtual object 210 and the glenoid projection virtual object 234, such as 40 or 50%, and may be automatically determined by the controller 110 or manually determined by the user 116. The overlap threshold may be a standard value determined from statistical shape models or otherwise may be patient specific. It will be understood that there are many variations of the overlap threshold, and those not specifically disclosed herein may be utilized. This would be an alternative to counting the numbers of discrete sections of the clockface system that is contemplated with reference to FIGS. 18 and 19.

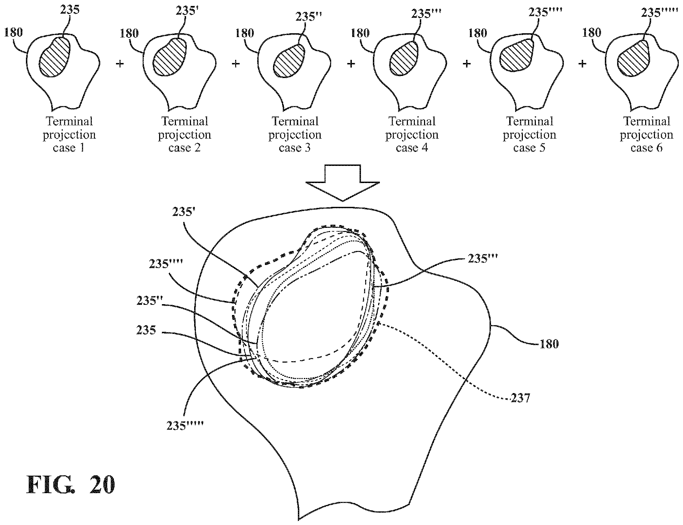

With reference to FIG. 20, it may be useful to visualize a glenoid projection virtual object 234 for the patient relative to non-patient glenoid projection data, such as non-patient glenoid projection virtual objects 235-235‴ʺ, 237 to help a user understand potential surgical interventions for the patient. The non-patient glenoid projection virtual objects 235-235‴ʺ, 237 may be collectively referred to as a non-patient glenoid projection virtual objects.

Similar to what is described for the glenoid projection virtual object 234 above, the non-patient glenoid projection virtual object(s) 235-235‴ʺ, 237 may be based on a terminal position(s) of the humerus and scapula of one or more persons other than the patient across various kinematic motions. In FIG. 20, these non-patient glenoid projection virtual object(s) 235-235‴ʺ, 237 are superimposed onto the humerus 3D model 180 of the patient to provide useful context for the planning of the intervention for the patient.

Different sources of the data associated with the terminal position of the humerus and scapula of one or more persons other than the patient are contemplated. In one example, the data associated with the terminal position of the humerus and scapula for the non-patient persons can be derived from the motion data described above that includes kinematic motions that include translation and rotation trajectories for the humerus and scapula of the population of persons. Again, as described above, the motion data may include measured values that may be based on kinematic measurements of a population of persons, including both uninjured and injured persons, and may be characterized as generalized motion data similar to what is described above for the patient motion simulation.

In order to accurately superimpose the non-patient glenoid projection virtual objects 235-235‴ʺ, 237 relative to patient's anatomy, and more specifically to the humerus 3D model 180, the controller may be configured to register the coordinate system of the non-patient motion data with the coordinate system of the patient data, such as the local humerus coordinate system 222 and local scapula coordinate system 224 described above. By registering the coordinate systems of the motion data with one or more of the humerus 3D model 180 and the scapula 3D model 190 of the patient, the non-patient glenoid projection virtual objects 235-235‴ʺ, 237 can be positioned and oriented in a way that is understandable to the user. More particularly, the coordinate system of the motion data can registered to one of the humerus 3D model 180 and the scapula 3D model 190, the non-patient glenoid projection virtual objects 235-235‴ʺ, 237 based on a technique that utilizes a statistical shape model or utilizes the dimensions of the fitted humerus 3D model 180 and the fitted scapula 3D model 190.

Various configurations of the non-patient glenoid projection virtual object 235-235‴ʺ, 237 are contemplated. In one example, the non-patient glenoid projection virtual object 235-235‴ʺ, 237 is based on a terminal position of a humerus relative to a scapula for each person of a plurality of non-patient persons 235-235‴ʺ. Thus, a user would be able to visualize the patient's glenoid projection relative to the glenoid projection of two or more other persons other than the patient. In this configuration, the non-patient virtual object of two or more other persons is based on a first non-patient virtual object 235 of a first non-patient and a second non-patient virtual object 235' of a second non-patient. The first non-patient virtual object 235 will be derived from the terminal position of the humerus relative to the scapula for a first non-patient person. The second non-patient virtual object 235' will be derived from the terminal position of the humerus relative to the scapula for a second non-patient person. The controller 110 may be configured to display the glenoid projection virtual object 234 for the patient along with the non-patient virtual objects 235, 235' associated with the first non-patient person and the second non-patient person relative to the humerus 3D model 180.

In instances where the non-patient glenoid project virtual objects 235-235‴ʺ, 237 are based on a terminal position for a plurality of persons, it is likely that the virtual objects associated with the different non-patient persons may overlap with one another. In instances where the method includes virtual objects associated with glenoid projections of a plurality of non-patient persons, the method may include determining an aggregate non-patient glenoid projection virtual object 237 having an area that matches the area of all of the glenoid projection virtual objects 235-235‴ʺ combined. In other words, the area encompassed by aggregate non-patient glenoid projection virtual object 237 is the cumulative area encompassed by a plurality of the non-patient glenoid projection virtual objects 235-235‴ʺ merged together. The aggregate non-patient glenoid projection virtual object 237 may define a boundary.

In FIG. 20, the dashed line of the aggregate non-patient glenoid projection virtual object 237 represents the boundary of the projections of the glenoid based on the population of patients that were studied. While described above as being based on all of the projections of the studied non-patient projections, alternatively it could be based on a statistical representation of all the non-patient glenoid projections, such as an average or within a given number of standard deviations, etc. from the studied non-patient glenoid projections.

In these instances, the rendering of the aggregate non-patient glenoid projection virtual object 237 may be based on a density of overlap of any particular portion of the projection from the underlying non-patient glenoid projection virtual objects 235-235‴ʺ. If there is a higher density of overlap of the non-patient glenoid projection virtual objects for a particular portion, that portion of the aggregate non-patient glenoid projection virtual object 237 may be rendered with a darker color than for a portion of the aggregate non-patient glenoid projection virtual object 237 that has a lower density of overlap of the various non-patient glenoid projection virtual objects 235-235‴ʺ. The density may be determined by how many non-patient persons exhibit a glenoid projection in a particular portion of the aggregate non-patient glenoid projection virtual object 237. For example, if a particular portion of the aggregate non-patient glenoid projection virtual object 237 is derived from the glenoid projections overlapping with the projections of two non-patient persons, the overlapping portion of the aggregate non-patient projection virtual object 237 will be a first color. If a particular portion of the aggregate non-patient glenoid projection is derived from the glenoid projections overlapping with the projections of three non-patient persons, the overlapping portion will be a second color, different from the first color. Alternatively, different densities of overlap underlying the aggregate non-patient glenoid projection virtual object 237 may be illustrated in other ways beyond color selection, such as different degrees of transparency, different shades of a color (light blue, medium blue, dark blue), different color intensities (e.g., dark grey, light grey, or black) or with a particular pattern.

In the example illustrated in Fig. 20, the renderings of the non-patient person glenoid projection virtual objects 235-235‴ʺ were derived from the terminal positions of six different patients. It should be understood that while this example includes the virtual objects corresponding to the terminal positions for six non-patient persons, any number of non-patient glenoid projection virtual objects for any number of non-patient persons may be visualized relative to the patient's humerus 3D model 180.

Referring to FIG. 21, the method may include rendering a lesion virtual object 210' relative to the humerus 3D model 180 with the glenoid projection virtual object 234, the non-patient glenoid project virtual objects 235-235‴ʺ, 237, or combinations thereof. While the aggregate non-patient glenoid projection virtual object 237 is depicted here, it is contemplated that other non-patient glenoid projection virtual objects 235-235‴ʺ may also be displayed.

The lesion virtual object 210' is shown as overlapping the glenoid projection virtual object 234 and the aggregate non-patient glenoid projection virtual object 237. The controller 110 may assess the state of a glenohumeral joint, i.e., track engagement, based on the location of the lesion virtual object 210 relative to the glenoid projection virtual object 234, such as how much area overlaps between the two objects, or whether any portion of the lesion virtual object 210' is outside of the glenoid projection virtual object 234. The controller 110 may be configured to complete this assessment automatically or render the structures on the display 120 for the user 116 to manually determine the presence and/or degree of overlap between the lesion virtual object 210 and the glenoid projection virtual object 234. Furthermore, the controller 110 may be configured to control an indicator based on this assessment to indicate to the user whether the lesion virtual object 210' is on-track or off-track.

Referring to images on the right side of Fig. 21, the method may further include rendering a planned glenoid projection virtual object 239-239'' relative to one or more 3D models, such as the humerus 3D model 180, generated from a segmentation of image data of the patient shoulder anatomy. The planned glenoid projection virtual object 239-239" may be a representation of how the glenoid is intended to engage the scapula in the terminal position after the surgical intervention has been completed. The planned glenoid projection virtual object 239-239" may have the same dimensions as the glenoid projection virtual object 234 but has a different pose than the glenoid projection virtual object 234. The pose of the planned glenoid projection virtual object 239-239" may be adjusted by the user through interacting with the user input device described above, such as by manipulating various arrow icons rendered on the screen or by dragging the planned glenoid projection virtual object to a desired position.

The ability to position the planned glenoid projection virtual object 239-239" allows the surgeon to understand whether the joint would be likely to be on track or off track at the limits of external rotation at 90 degrees. The user may reposition the planned glenoid projection virtual object 239-239" using the GUI or other user input device based on perceived patient laxity or based on the particular activities that the patient is engaged in, such as the particular sport that the patient is engaged in. This visualization of the planned glenoid projection virtual object 239-239" relative to the lesion virtual object 210' may enable the user to determine the most appropriate surgical procedure for the patient, which may include a reconstructive option such as a bone block procedure (e.g., Latarjet), Bankart repair, remplissage, or a humeral bone graft procedure.

For example, if the patient is hyperflexible (lax), the user could position the planned glenoid projection virtual object 239-239" at a position that would be representative of someone with greater laxity and see if the lesion virtual object 210' is on-track or off-track. Likewise, if the patient is stiff, the user can position the planned glenoid projection virtual object 239-239" in a less extreme position and evaluate whether the lesion virtual object 210' is on-track or off-track. Furthermore, by virtue of displaying the aggregate non-patient glenoid projection virtual object 237 in conjunction with the planned glenoid projection virtual object 239-239", the surgeon could see whether the adjusted position of the planned glenoid projection virtual object 239-239" is reasonable by comparing the adjusted position of the planned glenoid projection virtual object 239-239" to the aggregate non-patient projection virtual object 237.

The glenoid projection virtual object 234 may be rendered differently from the planned glenoid projection virtual object 239-239" so that these virtual objects may be easily distinguished from one another. For example, the glenoid projection virtual object 234 may be rendered in a first color and the planned glenoid virtual object 239-239" may be rendered in a second color, different from the first color. Alternatively, the glenoid projection virtual object 234 may be rendered in a pattern different from the planned glenoid projection virtual object 239-239". In one potential implementation, the controller 110 may be configured to assess the track engagement based on the planned glenoid projection virtual object 239-239" and the lesion virtual object 210' in the same manner as described above with respect to the glenoid projection virtual object 234, e.g., based on the area of overlap between the planned glenoid projection virtual object 239-239" and the lesion virtual object 210', using the clockface system as described above with respect to FIGS. 18 and 19, i.e., counting segments or overlap, etc. Of course, it is contemplated that the track engagement could be determined in other ways as well.

The method may further include providing a margin of deviation indicator based on the planned glenoid project virtual object and the aggregate glenoid projection virtual object. More specifically, the method may provide a margin of deviation indicator based on the boundary of the aggregate non-patient glenoid projection virtual object 237 and the planned glenoid projection virtual object 239-239". For example, on the uppermost right-hand image in Fig. 21, the planned glenoid projection virtual object 239 is wholly within the boundary of the aggregate non-patient glenoid projection virtual object 237. In these circumstances, the margin of deviation indicator would indicate that the adjusted terminal position is within the common boundary, such as displaying text "adjusted terminal glenoid position is inside the common boundary" on the display 120. The margin of deviation indicator may include rendering the planned glenoid projection virtual object 239 a first color, such as green, if the planned glenoid projection virtual object 239 is wholly within the common boundary of the aggregate non-patient glenoid projection virtual object 237.

With respect to the middle right hand image in Fig. 21, the planned glenoid projection virtual object 239' is at least partially outside of the boundary of the aggregate non-patient glenoid projection virtual object 237. The controller 110 may control the margin of deviation indicator to indicate the extent by which the planned glenoid projection virtual object 239' is outside of the boundary of the aggregate non-patient glenoid projection virtual object 237. For example, if the planned glenoid projection virtual object 239' positioned is outside of the common boundary by a first amount or less, the margin of deviation indicator may display text that indicates the extent that the adjusted glenoid position is outside the boundary, such as 10 % of the planned glenoid projection virtual object 239' is outside of the common boundary. In one approach, the margin of deviation is determined by counting the mm² of all triangles of the planned glenoid projection virtual object 239' that is outside of the common boundary defined by the aggregate non-patient glenoid projection virtual object 237 divided by the count of all of the mm² of all the triangles of the planned glenoid projection virtual object 239'. The margin of deviation indicator may include rendering the planned glenoid projection virtual object 239' in a second color, such as orange, so long as the extent is below a first threshold percentage, or rendering the portion of the glenoid projection virtual object 239' that is outside of the aggregate non-patient glenoid projection virtual object 237 differently than the aggregate non-patient glenoid projection virtual object 237, such as with a different color, with a different opacity, different color shade, or a different pattern..

With respect to the bottom right-hand image in Fig. 21, the planned glenoid projection virtual object 239" is at least partially outside of the boundary of the aggregate non-patient glenoid projection virtual object 237. If the planned glenoid projection virtual object 239" is outside of the common boundary defined by the aggregate non-patient glenoid projection virtual object 237 by a second threshold percentage, the margin of deviation indicator may indicate the extent that the adjusted glenoid position is outside the boundary, such as by displaying text that indicates the extent that the planned glenoid projection virtual object 239" is outside the boundary, such as 30 % of the planned glenoid projection virtual object 239' is outside of the common boundary defined by the aggregate non-patient glenoid projection virtual object 237. The margin of deviation indicator may include rendering the planned glenoid projection virtual object 239" in a third color, such as red, so long as the extent is above the second threshold percentage. In other words, the margin of deviation indicator may be based on a boundary of the planned glenoid projection virtual objects 239-239" and the boundary of the aggregate non-patient glenoid projection virtual object 237.

While it is described and shown that the margin of deviation indicator may be implemented with text and with colorization of the planned glenoid virtual object 239-239", it should be appreciated that the margin of deviation indicator may be implemented in other ways, such as by colorizing other aspects of the GUI, including colorizing other virtual objects, providing alert icons and/or symbols, or by providing colored text.

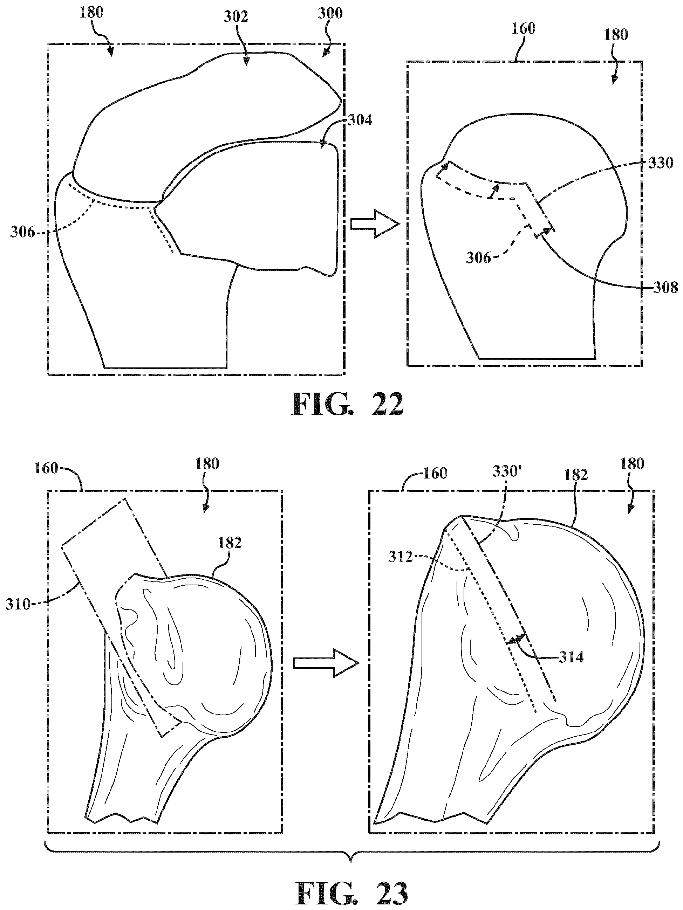

Referring to FIGS. 22-25, the controller 110 may utilize soft tissue information to facilitate display of information that will allow a user to better understand potential surgical interventions. Referring to FIG. 22, the controller 110 or other computing device may employ a segmentation algorithm to segment image data of a rotator cuff muscles of the patient 112 (such as a supraspinatus and an infraspinatus) and generate 3D models, which include a rotator cuff model 300, a supraspinatus 3D model 302 and an infraspinatus 3D model 304. Other muscles may also be found in the image data of the patient 112, and those not disclosed herein may be utilized in addition to the supraspinatus and the infraspinatus. With respect to the left-hand image of Figure 22, the segmentation algorithm may detect a lateral insertion location 306 of a tendon based on the positions of the supraspinatus 3D model 302 and the infraspinatus 3D model 304 relative to the humerus 3D model 180. The lateral insertion location 306 may represent the attachment location of the muscles of a rotator cuff to a humerus. The image data may be obtained by the imaging system 130, which may generate 3D CT image data, 3D MRI image data, or any other type of medical imaging data not disclosed herein. The landmarks, such as the soft tissue insertion location, may be represented by the landmarks stored in the statistical shape model, which become patient-specific by virtue of the statistical shape model being fit to the image data of the patient in question.

With respect to the right image of FIG. 22, the lateral insertion location 306 may optionally be translated a distance 308 based on the anatomical average of soft tissue attachment points in a medial or by a fixed threshold in order to generate insertion virtual object 330. This is because the medial insertion location of the rotator cuff is more pertinent to understanding track engagement than the lateral insertion location represented by lateral insertion location 306.

Referring to FIG. 23, in another aspect, the controller 110 may be configured to identify the location of one or more anatomical landmarks using curvature analysis. Curvature analysis may refer to an implementation that includes determining a plane 310 or line 312 which represents the area of greatest curvature of the humeral head portion 182, such as having the highest average curvature value. Anatomically, the plane 310 or line 312 representing the area of greatest curvature on the humeral head portion 182 may also be chosen to represent the most lateral insertion point of a rotator cuff. As such, the controller 110 may be further configured to adjust the plane 310 by an adjustment value 314 to represent the medial insertion of a rotator cuff or anatomical locations representing the attachment of the muscles of a rotator cuff. The adjustment value 314, such as the distance, from the most lateral insertion point (the plane 310 or line 312 to the insertion virtual object 330') may be based on common anatomical features, or otherwise determined for each patient 112, such as being based on a value indexed to the patient using various patient landmarks. After adjustment, the soft tissue line is depicted as 330'.

Other objects are also contemplated for representing the area of greatest curvature other than the plane on the humeral head portion 182, such as a line or other shape, and those not specifically described herein may be utilized. This method may be generally understood to include determining the location of at least one insertion point of the soft tissue corresponding to the attachment of soft tissue to the bones of the glenohumeral joint may include applying a machine learning model to the image data of the patient shoulder anatomy, applying curvature analysis to the image data of the patient shoulder anatomy, and fitting a plane based on a head of the humerus 3D model and a distance derived from a radius of the head of the humerus 3D model.

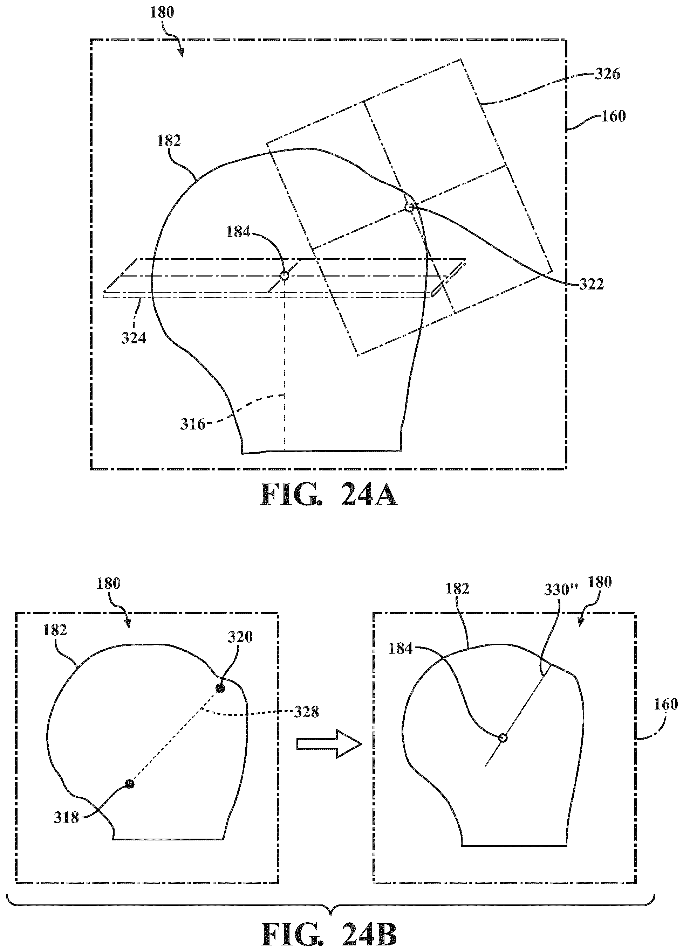

Another approach to identifying the location of the insertion of a rotator cuff is shown in FIGS. 24A and 24B. In this approach, the controller 110 may be configured to identify one or more anatomical locations including a humeral head center, a humeral shaft axis, an intertubercular sulcus, a rotator cable, and/or a lateral most point on the tuberculum majoris using the segmentation techniques described throughout. Other anatomical locations may be identified that are not disclosed here, and these anatomical locations may be represented by a virtual point, line, or other objects not disclosed herein. For example, the controller 110 may be configured to generate a humeral head center point 184, a humeral shaft axis object 316, an intertubercular sulcus point 318, a rotator cable point 320, and/or a lateral most point 322 on the tuberculum majoris and display these virtual objects in relation to the renderings of the 3D models. The controller 110 may be further configured to generate an axial plane 324 based on the humeral head center point 184 and/or a coronal plane 326 based on the lateral most point 322. The axial plane 324 may be based on the humeral shaft axis object 316. In some implementations, the axial plane 324 may be perpendicular to the humeral shaft axis object 316 and include the humeral head center point 184. In some implementations, the axial plane 324 may be differently related to the humeral shaft axis object 316, or even unrelated to the humeral shaft axis object 316. The coronal plane 326 may be based on the axial plane 324. In some implementations, the coronal plane 326 may be perpendicular to the axial plane 324 and include the lateral most point 322.

The controller 110 may be configured to determine a relationship between the location of the intertubercular sulcus point 318 and the location of the rotator cable point 320. The relationship may be represented as a line, a plane, or any other shape, and may be projected to represent the insertion of a rotator cuff. The projection direction of the relationship may be based upon the coronal plane 326, for example, being perpendicular to the coronal plane 326. In one implementation, the relationship is represented by a plane 328, which contains the intertubercular sulcus point 318 and the location of the rotator cable point 320. The plane 328 is perpendicular to the coronal plane 326 and is shifted to include the humeral head center point 184. The controller 110 may be configured to generate an insertion virtual object 330" (referred to below as the soft tissue insertion line 330) that intersects the humeral head center point 184 and the plane 328, the insertion virtual object 330" representing the insertion of a rotator cuff. Other anatomical relationships based upon the coronal plane 326, the axial plane 324, and/or other anatomical features disclosed above may exist, and those not specifically disclosed herein may be utilized by the controller 110 to determine the location of the insertion of a rotator cuff. While it is contemplated that the controller 110 is configured to utilize the points and planes described above (rotator cable point 320, plane 328, etc.) and cause these objects to be displayed, in other implementations, the controller 110 may simply use these points and planes, and yet not display those points and planes.

As mentioned above, the controller 110 may be further configured to generate the soft tissue insertion line 330 representing the one or more anatomical landmarks and/or the location of the insertion of a rotator cuff. The soft tissue insertion line 330 may alternatively be generated as a plane or any other geometrical shape to represent the lateral border 175 of the glenoid track virtual object 170. For example, as shown in FIG. 25, the display may be configured to show the soft tissue insertion line 330 in relation to the humerus 3D model 180 of the patient shoulder anatomy. Further, the controller 110 may be configured to cause the display to show the soft tissue insertion line 330 on the surface of the humeral head portion 146 as a plane or other shape representing the location of the insertion of a rotator cuff. The soft tissue insertion line 330 may also be manipulated and/or adjusted to better reflect patient shoulder anatomy by the user 116 via the user input device 122. For example, the user 116 may touch the display 120 and drag the soft tissue insertion line 330 to a desired location.

Alternatively to the approaches described above, the controller 110 may determine one or more such locations, or locations of other soft tissue attachment points, for non-patient persons and compute an anatomical average between those locations to generate a virtual object on the humerus 3D model representing the insertion location of the tendon. The anatomical average represents an estimation of the attachment of a rotator cuff to a humerus determined by the controller 110.

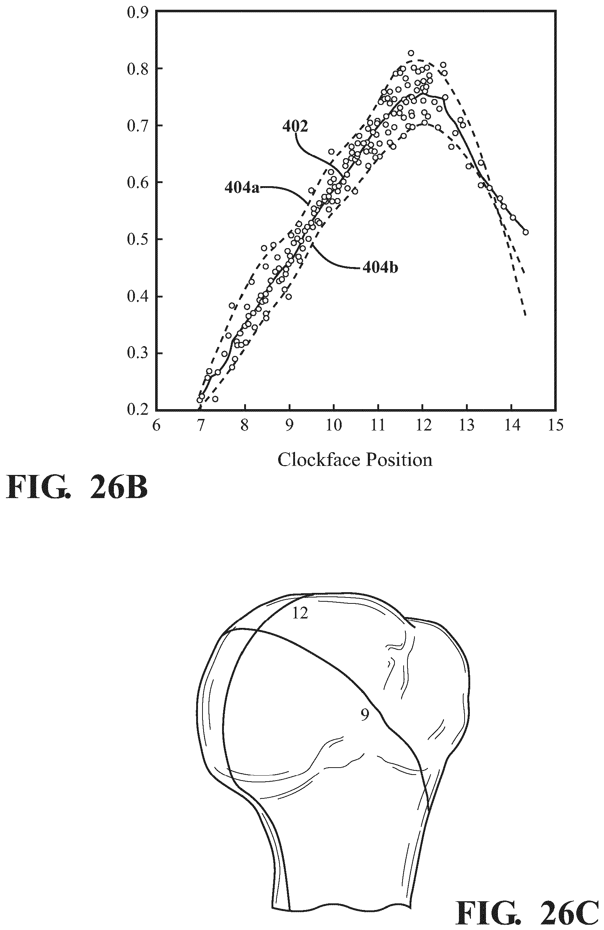

For example, with reference to FIGS. 26A-26C, in yet another configuration, the soft tissue insertion line for the patient may be based on collecting data for a plurality of non-patient persons. With reference to FIG. 26A, to collect such data, a plurality of radiopaque beads may be placed on the humerus for each person of the plurality of the non-patient persons. In particular, the plurality of radiopaque beads are spaced about the humerus along the medial insertion line of the rotator cuff according to slightly varying clockface positions. The clockface positions for the humerus are shown in FIG. 26C, with the clockface positions of 12 and 9 being seen in the provided view.

After placement of the plurality of beads on each non-patient person, each non-patient person is imaged. Then, the medial-lateral position of the rotator cuff insertion for each bead for each non-patient person is determined for each bead at each of the various clockface positions. The more medially placed beads may annotate the articular margin for each of the non-patient persons. The plot of these medial-lateral positions (plotted as a percentage of medial-lateral width) is depicted in FIG. 26B for each bead for each person for plurality of non-patient persons.

The line 402 in FIG. 26B shows the average medial-lateral position of the rotator cuff insertion over the plurality of non-patient persons for each clockface position. The line 404a represents an upper confidence bound and line 404b represents a lower confidence bound). The values of the line 402 or similar regression may be used to obtain the position of the soft tissue insertion line for the patient's specific anatomy, in instances where no radiopaque beads have been placed). By determining the medial-lateral width for a particular patient at each clockface position, a user can determine the medial insertion line (referred to as the insertion virtual object) for that particular patient. This insertion virtual object can be used to further plan the appropriate surgical approach as will be described below.

More particularly, as described above, this patient-specific line may be described as the soft tissue insertion line or insertion virtual object described above and is representative of the rotator cuff insertion. It may be rendered on the image of the patient anatomy, and may be registered to the patient's humerus 3D model, the scapula 3D model, or combination thereof using known techniques, such as by using shape models. Thus, this method may include receiving an average rotational insertion data set and registering the average rotational insertion data to the patient-specific humerus 3D model to represent a patient specific insertion virtual object representative of the likely position of the rotator cuff insertion. In summary, the position of the insertion virtual object described above may be determined using this approach as opposed to using the other approaches described above.

As described above and with reference to FIG. 25, it may be useful to visualize a glenoid projection virtual object 234 for the patient's humerus 3D model 180. It may also be useful to visualize the glenoid projection for the patient relative to the virtual object based on the insertion point of the soft tissue to help a user understand potential surgical interventions for the patient. As illustrated in FIG. 25, the method may include displaying at least a portion of a rendering of the humerus 3D model 180, a rendering of an insertion virtual object 330 based on the insertion point of the soft tissue, such as the rotator cuff, and a rendering based on the glenoid projection virtual object 234. In addition, a planned glenoid projection virtual object 239‴-239‴ʺ may also be displayed in addition to the glenoid projection virtual object 234 or as an alternative to the glenoid projection virtual object 234, as depicted in the images on the righthand side of FIG. 25.

With respect to the image on the lefthand side of FIG. 25, the track engagement of the patient's joint may be determined based on the insertion virtual object 330 and the lesion virtual object 210'. More specifically, the track engagement of the patient's joint, i.e., whether the lesion virtual object 210' is on-track or off-track, may be based on a distance from soft tissue insertion virtual object 330 to the medial border of the lesion virtual object 210'. If this distance is more than 83% of the healthy width of the glenoid, then controller 110 may designate this lesion as off-track. In addition, in an exemplary embodiment, if there is bone loss present, the amount of bone loss can be subtracted from the healthy width amount before comparing against the determined distance.

The healthy width can be determined by applying a surface prediction algorithm to the patient image data and/or the 3D model of patient shoulder anatomy. For example, the controller 110 may deploy a statistical shape model to generate a predicted surface of a healthy humeral head. More specifically, the controller 110 may be configured to fit a statistical shape model to the image data of patient shoulder anatomy to generate a premorbid prediction of the surface of the humeral head, i.e., a prediction of the surface of the humeral head before the occurrence of a pathology. Alternatively, the segmentation algorithm described above may be utilized to generate a surface based on a humeral mesh. This pre-morbid surface may be used to calculate the healthy width.

Referring to the images on the righthand side of FIG. 25, the method may include rendering an insertion virtual object 330 and a planned glenoid projection virtual object 239‴-239‴ʺ. More specifically, the method may provide an insertion indicator based on the position of the insertion virtual object 330 and a position the planned glenoid projection virtual object 239‴-239‴ʺ. More particularly, with respect to the uppermost righthand image of FIG. 25, a portion of an outer boundary of the planned glenoid projection virtual object 239‴ is spaced apart medially from the insertion virtual object 330. In this situation, the controller 110 may provide the insertion indicator by rendering the planned glenoid projection virtual object 239‴ a first color, such as green, because the outer boundary of the planned glenoid projection virtual object 239‴ is spaced medially from (and does not intersect with) the insertion virtual object 330.

In another example, with respect to the middle righthand image of FIG. 25, the planned glenoid projection virtual object 239ʺʺ intersects with the insertion virtual object 330, and as a result, a portion of an outer boundary of the planned glenoid projection virtual object 239ʺʺ is spaced laterally with respect to the insertion virtual object 330. In these circumstances, the controller 110 may provide the insertion indicator by rendering the planned glenoid projection virtual object 239ʺʺ in a second color, such as orange. Alternatively, the controller 110 may provide the insertion indicator by indicating the distance that the portion of the outer boundary of the adjusted position of the planned glenoid projection virtual object 239ʺʺ is spaced from the insertion object 330, such as by displaying text on the display 120 that indicates that the insertion object is spaced 4 mm from the planned glenoid projection virtual object 239ʺʺ.

In another example, with respect to the lowermost righthand image of FIG. 25, the planned glenoid projection virtual object 239‴ʺ intersects with the insertion virtual object 330, and a portion of an outer boundary of the planned glenoid projection virtual object 239‴ʺ is spaced apart laterally from the insertion virtual object 330. If the adjusted position of the planned glenoid projection virtual object 239‴ʺ is positioned such that the outer boundary of the planned glenoid projection virtual object 239‴ʺ is lateral to the insertion virtual object 330 by a second threshold amount, the controller 110 may provide the insertion indicator by displaying text that indicates the extent that the outer boundary of adjusted position of the planned glenoid projection virtual object‴ʺ is spaced from the insertion virtual object 330, such as by displaying text on the display 120 that indicates that the insertion object 330 is spaced 10 mm from the planned glenoid projection virtual object 239‴ʺ. The controller 110 may provide the insertion indicator by rendering the planned glenoid projection virtual object 239‴ʺ in a third color, such as red, when the outer boundary of the adjusted position of the planned glenoid projection virtual object 239‴ʺ is spaced from the insertion virtual object 330 by an amount that is above the second threshold, such as being spaced from the insertion virtual object 330 by more than 8 mm.

In the examples above, the computation regarding the amount that the outer boundary of the planned glenoid projection virtual object is spaced from the insertion virtual object 330 is based on the maximum amount that a portion of the boundary of the planned glenoid projection virtual object 239‴ʺ extends beyond the insertion virtual object 330 in any one direction. More particularly, in the illustrated configurations, the amount is calculated based on the maximum distance from the intersecting portion of the planned glenoid projection virtual object and the insertion virtual object 330 to the furthest point of the planned glenoid projection virtual object from the intersecting portion. However, it should be appreciated that the amount that the portion of the outer boundary of the planned glenoid projection extends beyond the insertion object 330 can be calculated in alternative ways.

It should be appreciated that the lesion, such as lesion virtual object 210' depicted in FIG. 21, can be illustrated in conjunction with the insertion virtual object 330 in the depictions shown in FIG. 25.

In addition, or as an alternative to the one or more features described above, the controller 110 may be configured to calculate and display information regarding bone blocks to facilitate surgical planning, particularly with respect to determining the appropriate placement of the planned glenoid projection virtual objects described above. The user may select a set of dimensions for a bone block object and visualize the bone block object to plan the appropriate reconstruction that may be necessary for the patient shoulder anatomy, such as the dimensions necessary to ensure that the lesions described throughout may be considered as an on-track lesion(s) after placement of the bone block for various planned glenoid projection virtual object positions.

The controller 110 may be configured to cause the display 120 to show renderings of the humerus and scapula 3D models 180,190 and any of the above-described anatomical characteristics or virtual objects relative to the renderings of the humerus and scapula 3D models 180, 190. These features may be displayed individually or in combination with each other. The GUI 160 may be configured for the user 116 to select what is shown on the display 120, and all possible configurations of the anatomical features determined by the controller 110 may be shown on the display 120. For example, the glenoid projection virtual object 234, the glenoid track virtual object 170, and the glenoid track line 172 may all be rendered on the display 120 in relation to renderings of the humerus 3D model 180. This disclosure is not meant to limit the system 100 to any combination of display configurations, and any of the described display configurations may be combined with other possible configurations. Similarly, the GUI 160 may include any of the above-described elements or any of the views presented in the Figures, including poses of the humerus 3D model 180 in relation to the scapula 3D model 190 and associated motion data applied to a glenohumeral joint. The GUI 160 may show a multitude of the views at once, or any combination of the views presented in the Figures. For example, the GUI 160 may be split into two or more windows, one window showing the humerus 3D model 180 and the glenoid track virtual object 170 and the other window showing the scapula 3D model 190 and the glenoid projection virtual object 234. The multitude of windows of the GUI 160 may be shown on one display or multiple displays. It is to be further understood that the surgical planning system 100 may be utilized to evaluate many types of patient anatomy and is not limited to the exemplary configuration described herein.

For example, the controller 110 may be configured to identify anatomical deformations relating to the hip joint, including a cam deformity on the femoral head-neck junction or a pincer deformity on the acetabular rim, or other anatomical deformations not described herein. These deformities may lead to hip impingement. The controller 110 may be configured to receive 3D models of the femur and the pelvis or apply segmentation algorithms to image data of the patient hip anatomy to generate 3D models. Further, the controller 110 may be configured to apply other segmentation algorithms and/or other methods to the 3D models and/or the image data of the patient hip anatomy to identify the anatomical deformations described above. The controller 110 may be configured to cause the display 120 to show the 3D models of the femur and the pelvis relative to each other.

Another configuration of the motion data may be motion trajectories of the hip joint of the patient 112. The motion data may include kinematic movements of the femur relative to the pelvis or the pelvis relative to the femur. For example, the motion trajectory may be indicative of deep flexion and/or a flexion adduction internal rotation motion. The motion data may include translational trajectories and/or rotational trajectories pertinent to the femur and/or the pelvis. In addition, the motion data may include aspects of kinematic movements such as shifting centers of rotation of the femur or the pelvis, pelvic inclination of an injured hip joint, and natural healthy motion. The controller 110 may be further configured to identify and align the local coordinate systems of the 3D models of the femur and the pelvis to a reference coordinate system based on the motion data. After aligning the coordinate systems, the controller 110 may be configured to apply the motion data to the 3D models of the femur and the pelvis and cause the display to show the poses of the models in accordance with the motion data. The user 116 may assess the state of the hip joint based on the visualization of the 3D models of the femur and the pelvis with motion data applied.

Alternatively, the controller 110 may automatically assess the state of the hip joint based on the 3D models of the femur and pelvis with the motion data applied. This automatic assessment may be configured to automatically determine a joint characteristic of the joint based on this information. The joint characteristic may include determining a joint engagement based on the applied motion data. The joint characteristic may include determining a factor indicative of joint impingement, such as the presence of the deformities described above, using this applied motion data, such as pelvic transverse plane range of motion.

The controller 110 may be configured to generate another visualization of the hip joint including a distance map comprising different colors on the surface of the femur and/or the pelvis in accordance with the proximity of anatomical deformations / features relative to other anatomical deformations / features. For each kinematic motion, a distance map may be generated with the proximity of the cam deformation and the pincer deformation represented on the 3D models of the femur and the pelvis. It is to be understood that the kinematic movements of the hip joint may include many motions not disclosed herein, and various configurations of the 3D models of hip models may be visualized on the display 120. The controller 110 may automatically assess the state of the hip joint based on the 3D models the distance map, such as determining a joint engagement based on distances below a certain threshold on the distance map.

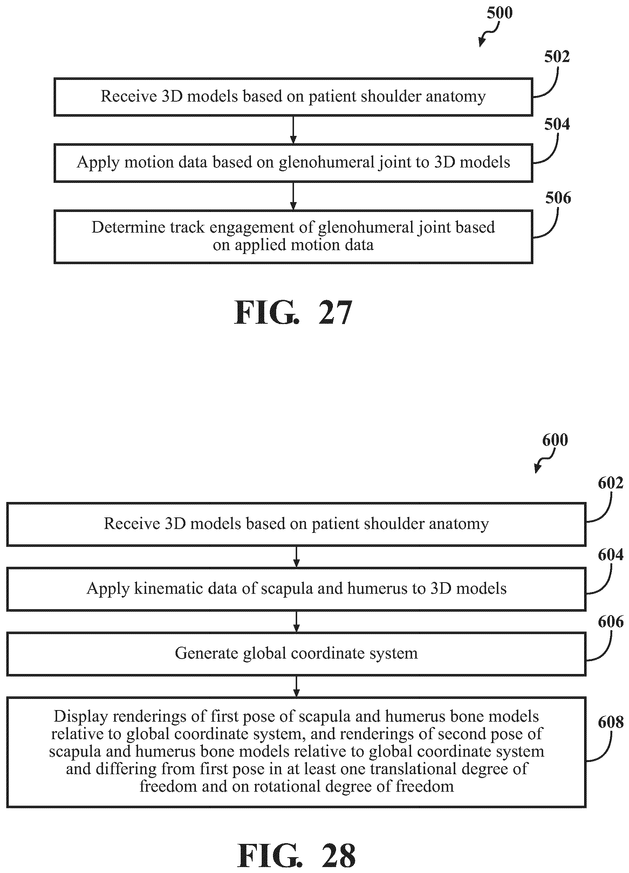

Having described the surgical planning system 100, a method 500 of assessing the patient shoulder anatomy using the surgical planning system 100 (i.e. the controller 110) is illustrated in FIG. 27. The method 500 may be executed pre-operatively to plan one or more shoulder surgeries for the patient 112. At step 502, the controller 110 may receive 3D models based on the patient shoulder anatomy. At step 504, the controller 110 may apply motion data based on a glenohumeral joint to the 3D models, as described above in section V. At step 506, the controller 110 may determine a track engagement of the glenohumeral joint based on the applied motion data, as described above in section VII.

A method 600 of visualizing the patient shoulder anatomy with the surgical system 100 is illustrated in FIG. 28. Again, the method 600 may be executed pre-operatively to plan one or more shoulder surgeries for the patient 112. According to some aspects, the controller 110 may receive a 3D dataset and generate the 3D model from the 3D dataset. At step 602, the controller 110 may receive 3D models of the patient shoulder anatomy, including a scapula 3D model 190 and a humerus 3D model 180, as shown in FIG. 2. At step 604, the controller 110 may apply kinematic data of a scapula and a humerus to the bone models 180, 190, as shown in FIGS. 3A-7B and described above in Section V. At step 606, the controller 110 may generate a global coordinate system 220, as shown in FIG. 10. At step 608, the controller 110 may display renderings of a first pose of the scapula 3D model 190 and/or the humerus 3D model 180 relative to the global coordinate system 220, and renderings of a second pose of the scapula 3D model 190 and/or the humerus 3D model 180 relative to the global coordinate system 220 and differing from the first pose in at least one translational degree of freedom and one rotational degree of freedom, as shown in FIGS. 3A-7B.

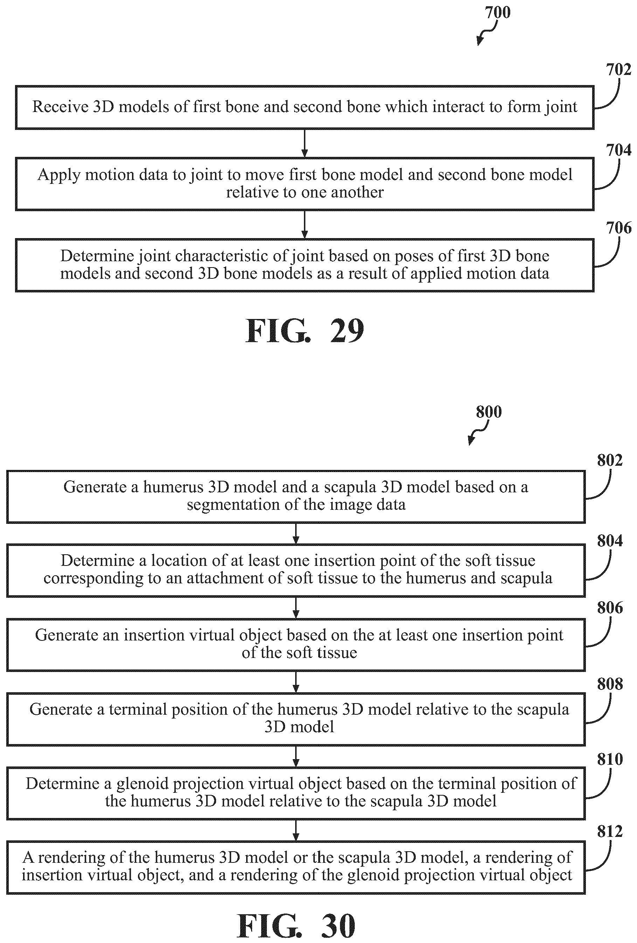

A method 700 of assessing a patient anatomy with the surgical system 100 is illustrated in FIG. 29. As with the previously described methods 500 and 600, the method 700 may be executed pre-operatively to plan one or more surgeries for the patient 112. According to some aspects, the controller 110 may receive a 3D dataset and generate the 3D model from the 3D dataset. Starting at step 702, the controller may receive 3D models of a first bone and a second bone which interact to form a joint. At step 704, the controller may apply motion data to the joint to move a first bone model and a second bone model relative to one another. The motion data may include a first pose of the first bone relative to the second bone and a second pose of the first bone relative to the second bone, where the first pose differs from the second pose in at least one translational degree of freedom and one rotational degree of freedom. At step 706, the controller may determine a joint characteristic of the joint based on poses of the first 3D bone models and the second 3D bone models as a result of the applied motion data. The method may further include displaying the determined joint characteristic.

A method 800 of assessing a patient anatomy with the surgical system 100 is illustrated in FIG. 30. As with the previously described methods, the method 800 may be executed pre-operatively to plan one or more surgeries for the patient 112. According to some aspects, the controller 110 may receive a 3D dataset and generate the 3D model from the dataset. Starting at step 802, the controller may generate a humerus 3D model and a scapula 3D model based on a segmentation of the image data. At step 804, the controller may determine a location of at least one insertion point of the soft tissue corresponding to an attachment of soft tissue to the humerus and scapula. At step 806, the controller may generate an insertion virtual object based on the at least one insertion point of the soft tissue. At step 808, the controller may generate a terminal position of the humerus 3D model relative to the scapula 3D model. At step 810, the controller may determine a glenoid projection virtual object based on the terminal position of the humerus 3D model relative to the scapula 3D model. At step 812, the controller may display a rendering of the humerus 3D model or the scapula 3D model, a rendering of insertion virtual object, and a rendering of the glenoid projection virtual object. In certain aspects, the controller 110 may perform the segmentation of the image data.

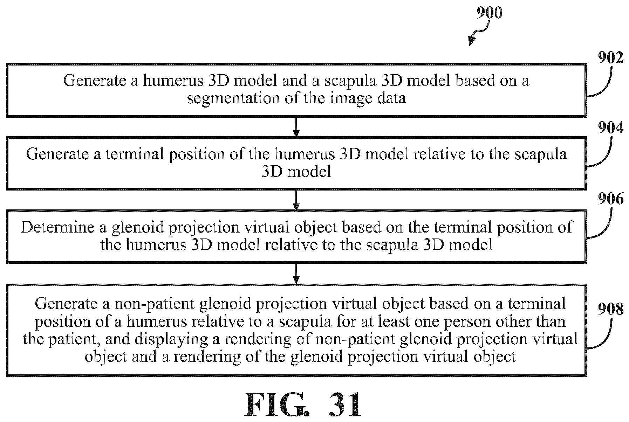

A method 900 of assessing a patient anatomy with the surgical system 100 is illustrated in FIG.31. As with the previously described methods, the method 900 may be executed pre-operatively to plan one or more surgeries for the patient 112. According to some aspects, the controller 110 may receive a 3D dataset and generate the 3D model from the dataset. At step 902, the controller may generate a humerus 3D model and a scapula 3D model based on a segmentation of the image data. At step 904, the controller may generate a terminal position of the humerus 3D model relative to the scapula 3D model. At step 906, the controller may determine a glenoid projection virtual object based on the terminal position of the humerus 3D model relative to the scapula 3D model. At step 908, the controller may generate a non-patient glenoid projection virtual object based on a terminal position of a humerus relative to a scapula for at least one person other than the patient, and display a rendering of non-patient glenoid projection virtual object and a rendering of the glenoid projection virtual object. In certain aspects, the controller 110 may perform the segmentation of the image data.

### CLAUSES FOR ADDITIONAL PROTECTION

I. A method of visualizing a patient shoulder anatomy, comprising: receiving one or more 3D models of the patient shoulder anatomy comprising a scapula 3D model and a humerus 3D model;
   applying kinematic data of a scapula and a humerus to the one or more 3D models, the kinematic data including: one or more translation trajectories of the scapula and the humerus relative to one another; and
   one or more rotation trajectories of the scapula and the humerus relative to one another; generating a global coordinate system; and displaying: renderings of a first pose of the scapula 3D model and the humerus 3D model relative to the global coordinate system; and renderings of a second pose of the scapula 3D model and the humerus 3D model relative to the global coordinate system and differing from the first pose in at least one translational degree of freedom and at least one rotational degree of freedom.
II. The method of clause I, wherein applying kinematic data of the scapula and the humerus to the one or more 3D models comprises generating a scapula coordinate system and a humerus coordinate system.
III. The method of clause II, wherein generating the humerus coordinate system comprises identifying anatomical features of interest of the humerus 3D model including one or more of:
   a humeral head center; a lateral epicondyle; a medial epicondyle; a humeral shaft axis; a greater tuberosity; and combinations thereof.
IV. The method of clause II or III, wherein generating the scapula coordinate system comprises identifying anatomical features of interest of the scapula 3D model including one or more of:
   a glenoid center; an inferior angle; a trigonum spinae; a posterior lateral acromion; an acromioclavicular joint; a scapular spine axis; a scapular wing plane; and combinations thereof.
V. The method of clause II, III, or IV, wherein the scapula coordinate system and the humerus coordinate system are aligned to the global coordinate system.
VI. The method of any one of clauses I to V, wherein displaying renderings of the scapula 3D model and the humerus 3D model comprises simultaneously displaying kinematics of the scapula 3D model and the humerus 3D model.
VII. The method of any one of clauses II-V, wherein displaying renderings of the scapula 3D model and the humerus 3D model comprises displaying the rendering of the scapula 3D model relative to the humerus coordinate system.
VIII. The method of any one of clauses II-VII, wherein displaying renderings of the scapula 3D model and the humerus 3D model comprises displaying the rendering of the humerus 3D model relative to the scapula coordinate system.
IX. A method of assessing a patient anatomy, comprising: receiving 3D models of a first bone and a second bone which interact to form a joint; applying motion data to the joint to move the first bone model and the second bone model relative to one another, wherein: the motion data comprises a first pose of the first bone relative to the second bone and a second pose of the first bone relative to the second bone; and the first pose differs from the second pose in at least one translational degree of freedom and one rotational degree of freedom; and determining a joint characteristic of the joint based on the poses of the first 3D bone model and the second 3D bone model as a result of the applied motion data.
X. The method of clause IX, further comprising displaying renderings of the first pose of the first 3D bone model relative to the second 3D bone model, and renderings of the second pose of the first 3D bone model relative to the second 3D bone model.
XI. The method of clause IX or X, wherein determining the joint characteristic of the joint based on the applied motion data comprises determining a joint engagement based on the applied motion data.
XII. The method of any one of clauses IX, X, or XI, wherein determining the joint characteristic of the joint based on the applied motion data comprises determining a factor indicative of joint impingement.
XIII. The method of clause XI or XII, wherein the motion data comprises a terminal position of the first bone relative to the second bone, wherein the step of determining joint engagement is based on the terminal position of the first bone relative to the second bone.
XIV. The method of clause XIII, wherein the terminal position of the first bone relative to the second bone comprises the maximum translational and rotational position of the first bone relative to the second bone.
XV. The method of clause XIV, further comprising displaying renderings of the first 3D bone model and the second 3D bone model in the terminal position.
XVI. A method of visualizing a patient shoulder anatomy, comprising: receiving image data of the patient shoulder anatomy comprising at least a portion of soft tissue, a humerus, and a scapula;
   generating a humerus 3D model and a scapula 3D model based on a segmentation of the image data; determining a location of at least one insertion point of the soft tissue corresponding to an attachment of soft tissue to the humerus and scapula; generating an insertion virtual object based on the at least one insertion point of the soft tissue; generating a terminal position of the humerus 3D model relative to the scapula 3D model; determining a glenoid projection virtual object based on the terminal position of the humerus 3D model relative to the scapula 3D model; and displaying: a rendering of the humerus 3D model or the scapula 3D model, a rendering of insertion virtual object, and a rendering of the glenoid projection virtual object.
XVII. The method of clause XVI, wherein: determining the location of at least one insertion point of the soft tissue corresponding to the attachment of soft tissue to the humerus and scapula comprises applying a machine learning model to the image data of the patient shoulder anatomy.
XVIII. The method of clause XVI or XVII, wherein: determining the location of at least one insertion point of the soft tissue corresponding to the attachment of soft tissue to the humerus and scapula comprises applying curvature analysis to the image data of the patient shoulder anatomy.
XIX. The method of any one of clauses XVI-XVIII, wherein the one or more 3D models comprises a humerus 3D model and wherein determining a location of at least one insertion point of the soft tissue corresponding to the attachment of soft tissue to the humerus and scapula comprises fitting a plane based on a head of the humerus 3D model and a distance derived from a radius of the head of the humerus 3D model.
XX. The method of any one of clauses XVI-XIX, further comprising the generating a non-patient glenoid projection virtual object based on a terminal position of a humerus relative to a scapula for each person of a population of persons, and wherein the method further comprises displaying the non-patient glenoid projection virtual object on the rendering of the humerus 3D model or the scapula 3D model.
XXI. The method of clause XX, wherein the non-patient glenoid project virtual object is registered to one of the humerus 3D model and the scapula 3D model based on a technique selected from a statistical shape model or a based on dimensions of the humerus 3D model and the scapula 3D model.
XXII. The method of any one of clauses XVI-XXI, further comprising determining an insertion indicator based on the insertion virtual object and the glenoid projection virtual object.
XXIII. The method of clause XXIII, wherein the insertion indicator is based on a boundary of the glenoid projection virtual object and the insertion virtual object.
XXIV. The method of any one of clauses XVI-XXIII, further comprising: receiving a pose of a planned glenoid projection virtual object, displaying a rendering of the planned glenoid projection virtual object, the planned glenoid projection virtual object having the same dimensions as the glenoid projection virtual object.
XXV. The method of clause XXIV, further comprising displaying the planned glenoid projection in a position different than a position of the glenoid projection virtual object.
XXVI. The method of clause XXIV or XXV, further comprising determining an insertion indicator based on the insertion virtual object and the pose of planned glenoid projection virtual object.
XXVII. The method of clause XXVI, wherein the insertion indicator is based on a boundary of the planned glenoid projection virtual object and the insertion virtual object.
XXVIII. A method of visualizing a patient shoulder anatomy, comprising: receiving image data of the patient shoulder anatomy comprising at least a portion of soft tissue, a humerus, and a scapula; generating a humerus 3D model and a scapula 3D model based on a segmentation of the image data; generating a terminal position of the humerus 3D model relative to the scapula 3D model; determining a glenoid projection virtual object based on the terminal position of the humerus 3D model relative to the scapula 3D model; generating a non-patient glenoid projection virtual object based on a terminal position of a humerus relative to a scapula for at least one person other than the patient, and displaying a rendering of non-patient glenoid projection virtual object and a rendering of the glenoid projection virtual object.
XXIX. The method of clause XXVIII, further comprising determining a margin of deviation based on the glenoid projection virtual object and the non-patient glenoid projection virtual object.
XXX. The method of clause XXIX, wherein the margin of deviation is based on a boundary of the glenoid projection virtual object and a boundary of the non-patient glenoid projection virtual object.
XXXI. The method of any one of clauses XXVIII-XXX, further comprising: receiving a pose of a planned glenoid projection virtual object, displaying a rendering the planned glenoid projection virtual object, the planned glenoid projection virtual object having the same dimensions as the glenoid projection virtual object.
XXXII. The method of clause XXXI, further comprising displaying the planned glenoid projection in a position different than a position of the glenoid projection virtual object.
XXXIII. The method of clause XXXI or XXXII, further comprising determining a margin of deviation based on the planned glenoid projection virtual object and the non-patient glenoid projection virtual object.
XXXIV. The method of clause XXXIII, wherein the margin of deviation is based on a boundary of the planned glenoid projection virtual object and a boundary of the non-patient glenoid projection virtual object.
XXXV. The method of any one of clauses XXVIII-XXXIV, wherein the non-patient virtual object is based on a terminal position of a humerus relative to a scapula for each person of a population of persons.
XXXVI. The method of clause XXXV, wherein the non-patient virtual object is registered to one of the humerus 3D model and the scapula 3D model based on a technique selected from a statistical shape model or a based dimensions of the humerus 3D model and the scapula 3D model.
XXXVII. The method of clause XXXV or XXXVI, wherein the rendering of the non-patient glenoid projection virtual object is based on a density of overlap of a glenoid projection of the population of persons for each portion of the glenoid projection on the humerus 3D model.
XXXVIII. The method of any one of clauses XXIX-XXXVII, wherein non-patient virtual object includes a first non-patient glenoid projection virtual object and a second non-patient glenoid projection virtual object, and wherein the method is further defined as displaying a rendering of the first non-patient glenoid projection virtual object and the second non-patient glenoid projection virtual object.
XXXIX. The method of clause XXXVIII, further comprising the generating the first non-patient glenoid projection virtual object based on a terminal position of a humerus relative to a scapula for a first person other than the patient, and generating the second non-patient glenoid projection virtual object based on a terminal position of a humerus relative to a scapula for a second person other than the patient.
XL. The method of any one of clauses XXXV-XXXIX, wherein non-patient glenoid projection virtual object is further defined as an aggregate of glenoid projections based on a terminal position of a humerus relative to a scapula for a plurality of persons other than the patient.
XLI. A non-transitory computer readable storage medium having stored therein data representing instructions executable by a programmed processor for assessing a patient shoulder anatomy, the storage medium comprising instructions for: receiving one or more 3D models based on the patient shoulder anatomy; applying motion data based on a glenohumeral joint to the one or more 3D models; and determining a track engagement of the glenohumeral joint based on the motion data.
XLII. A non-transitory computer readable storage medium having stored therein data representing instructions executable by a programmed processor for visualizing a patient shoulder anatomy, the storage medium comprising instructions for: receiving one or more 3D models based on the patient shoulder anatomy comprising a scapula 3D model and a humerus 3D model; applying kinematic data of a scapula and a humerus to the one or more 3D models, the kinematic data including: one or more translation trajectories of the scapula and the humerus relative to one another; and one or more rotation trajectories of the scapula and the humerus relative to one another; generating a global coordinate system; and displaying: renderings of a first pose of the scapula 3D model and the humerus 3D model relative to the global coordinate system; and renderings of a second pose of the scapula 3D model and the humerus 3D model relative to the global coordinate system and differing from the first pose in at least one translational degree of freedom and one rotational degree of freedom.
XLIII. A non-transitory computer readable storage medium having stored therein data representing instructions executable by a programmed processor for performing one or more of the steps of clauses I to VIII.
XLIV. A non-transitory computer readable storage medium having stored therein data representing instructions executable by a programmed processor for assessing a patient anatomy, the storage medium comprising instructions for: receiving 3D models of a first bone and a second bone which interact to form a joint; applying motion data to the joint to move the first bone model and the second bone model relative to one another, wherein: the motion data comprises a first pose of the first bone relative to the second bone and a second pose of the first bone relative to the second bone; and the first pose differs from the second pose in at least one translational degree of freedom and one rotational degree of freedom; and determining a joint characteristic of the joint based on the poses of the first 3D bone model and the second 3D bone model as a result of the applied motion data.
XLV. A non-transitory computer readable storage medium having stored therein data representing instructions executable by a programmed processor for performing one or more of the steps of clauses IX to XV.
XLVI. A non-transitory computer readable storage medium having stored therein data representing instructions executable by a programmed processor for assessing a patient shoulder anatomy, the storage medium comprising instructions for: receiving image data of the patient shoulder anatomy comprising at least a portion of soft tissue, a humerus, and a scapula; generating a humerus 3D model and a scapula 3D model based on a segmentation of the image data; determining a location of at least one insertion point of the soft tissue corresponding to an attachment of soft tissue to the humerus and scapula; generating an insertion virtual object based on the at least one insertion point of the soft tissue; generating a terminal position of the humerus 3D model relative to the scapula 3D model; determining a glenoid projection virtual object based on the terminal position of the humerus 3D model relative to the scapula 3D model; and displaying: a rendering of the humerus 3D model or the scapula 3D model, a rendering of insertion virtual object, and a rendering of the glenoid projection virtual object.
XLVII. A non-transitory computer readable storage medium having stored therein data representing instructions executable by a programmed processor for assessing a patient shoulder anatomy, the storage medium comprising instructions for: receiving image data of the patient shoulder anatomy comprising at least a portion of soft tissue, a humerus, and a scapula; generating a humerus 3D model and a scapula 3D model based on a segmentation of the image data; generating a terminal position of the humerus 3D model relative to the scapula 3D model; determining a glenoid projection virtual object based on the terminal position of the humerus 3D model relative to the scapula 3D model; generating a non-patient glenoid projection virtual object based on a terminal position of a humerus relative to a scapula for at least one person other than the patient, and displaying a rendering of non-patient glenoid projection virtual object and a rendering of the glenoid projection virtual object.
XLVIII. A computing system comprising: a memory configured to store image data of a patient shoulder anatomy; and a controller configured to perform the steps of clause **I,** and any clauses dependent thereon.
XLIX. A computing system comprising: a memory configured to store image data of a patient shoulder anatomy; and a controller configured to perform the steps of clause IX, and any clauses dependent thereon.
L. A computing system comprising: a memory configured to store image data of a patient shoulder anatomy; and a controller configured to perform the steps of clause XVI, and any clauses dependent thereon.
LI. A computing system comprising: a memory configured to store image data of a patient shoulder anatomy; and a controller configured to perform the steps of clause XXVIII, and any clauses dependent thereon.

Any of the above methods may be executed independently, or concurrently with the other disclosed methods, and any of the presented steps may be executed in any combination or order not disclosed herein. Any of the above methods may be carried out by the surgical planning system 100 to aid the user 116 in performing an assessment of the patient glenohumeral joint or other joints and/or determining the likelihood of a lesion having an impact on joint engagement.

Any of the above methods may be executed independently, or concurrently with the other disclosed methods, and any of the presented steps may be executed in any combination or order not disclosed herein. Any of the above methods may be carried out by the surgical planning system 100 to aid the user 116 in performing an assessment of the patient anatomy and/or visualizing the patient anatomy.

In one or more examples, the functions described may be implemented in hardware, software, firmware, or any combination thereof. If implemented in software, the functions may be stored on or transmitted over as one or more instructions or code on a computer-readable medium and executed by a hardware-based processing unit, such as controller 110. Computer-readable media may include computer-readable storage media, which corresponds to a tangible medium such as data storage media, or communication media including any medium that facilitates transfer of a computer program from one place to another, e.g., according to a communication protocol. In this manner, computer-readable media generally may correspond to (1) tangible computer-readable storage media which is non-transitory or (2) a communication medium such as a signal or carrier wave. Data storage media may be any available media that can be accessed by one or more computers or one or more processors to retrieve instructions, code and/or data structures for implementation of the techniques described in this disclosure. A computer program product may include a computer-readable medium.

By way of example, and not limitation, such computer-readable storage media can comprise RAM, ROM, EEPROM, CD-ROM or other optical disk storage, magnetic disk storage, or other magnetic storage devices, flash memory, or any other medium that can be used to store desired program code in the form of instructions or data structures and that can be accessed by a computer. Also, any connection is properly termed a computer-readable medium. For example, if instructions are transmitted from a website, server, or other remote source using a coaxial cable, fiber optic cable, twisted pair, digital subscriber line (DSL), or wireless technologies such as infrared, radio, and microwave, then the coaxial cable, fiber optic cable, twisted pair, DSL, or wireless technologies such as infrared, radio, and microwave are included in the definition of medium. Disk and disc, as used herein, includes compact disc (CD), laser disc, optical disc, digital versatile disc (DVD), floppy disk and Blu-ray disc, where disks usually reproduce data magnetically, while discs reproduce data optically with lasers. Combinations of the above should also be included within the scope of computer-readable media.

Operations described in this disclosure may be performed by one or more processors, which may be implemented as fixed-function processing circuits, programmable circuits, or combinations thereof, such as one or more digital signal processors (DSPs), general purpose microprocessors, application specific integrated circuits (ASICs), field programmable gate arrays (FPGAs), or other equivalent integrated or discrete logic circuitry. Fixed-function circuits refer to circuits that provide particular functionality and are preset on the operations that can be performed. Programmable circuits refer to circuits that can be programmed to perform various tasks and provide flexible functionality in the operations that can be performed. For instance, programmable circuits may execute instructions specified by software or firmware that cause the programmable circuits to operate in the manner defined by instructions of the software or firmware. Fixed-function circuits may execute software instructions (e.g., to receive parameters or output parameters), but the types of operations that the fixed-function circuits perform are generally immutable. Accordingly, the terms "processor" and "processing circuitry," as used herein may refer to any of the foregoing structures or any other structure suitable for implementation of the techniques described herein.

It will be further appreciated that the terms "include," "includes," and "including" have the same meaning as the terms "comprise," "comprises," and "comprising." Moreover, it will be appreciated that terms such as "first," "second," "third," and the like are used herein to differentiate certain structural features and components for the non-limiting, illustrative purposes of clarity and consistency. The terminology which has been used is intended to be in the nature of words of description rather than of limitation. Many modifications and variations are possible in light of the above teachings may be practiced otherwise than as specifically described.

Several implementations have been discussed in the foregoing description. However, the implementations discussed herein are not intended to be exhaustive or limit the invention to any particular form. The terminology which has been used is intended to be in the nature of words of description rather than of limitation. Many modifications and variations are possible in light of the above teachings and the invention may be practiced otherwise than as specifically described.

The many features and advantages of the invention are apparent from the detailed specification, and thus, it is intended by the appended claims to cover all such features and advantages of the invention which fall within the true spirit and scope of the invention. Further, since numerous modifications and variations will readily occur to those skilled in the art, it is not desired to limit the invention to the exact construction and operation illustrated and described, and accordingly, all suitable modifications and equivalents may be resorted to, falling within the scope of the invention.

## Claims

1. A method of determining a track engagement of a glenohumeral joint of a patient shoulder anatomy, comprising:
receiving one or more 3D models based on the patient shoulder anatomy;
applying motion data based on the glenohumeral joint to the one or more 3D models;
determining a track engagement of the glenohumeral joint based on the applied motion data; and
displaying an indicator based on the track engagement.

2. The method of claim 1, wherein receiving the one or more 3D models comprises generating one or more 3D models from patient image data of the patient shoulder anatomy and further comprises:
determining a surface model of the patient shoulder anatomy; and
applying the surface model of the patient shoulder anatomy to a statistical shape model.

3. The method of claim 2, wherein the one or more 3D models includes a humerus 3D model including a humeral head portion, the method further comprising identifying a lesion on the humeral head portion of the humerus 3D model based on the patient image data by:
providing at least a portion of the patient image data as an input to a deep learning network;
receiving a lesion virtual object as an output from the deep learning network; and
displaying a rendering of the lesion virtual object relative to a portion of a rendering of the one or more 3D models.

4. The method of any preceding claim, further comprising determining an impact rating representing a joint engagement based on the motion data; and wherein the indicator is based on the impact rating.

5. The method of any preceding claim, wherein the one or more 3D models comprise a humerus 3D model and a scapula 3D model and further comprising displaying renderings of a first pose of the humerus 3D model, a first pose of the scapula 3D model, a second pose of the humerus 3D model, and a second pose of the scapula 3D model in accordance with the motion data applied to the one or more 3D models.

6. The method of any preceding claim, wherein the motion data based on the glenohumeral joint comprises generalized motion data based on kinematic measurements of a population of persons having a joint including a scapula and a humerus, wherein the motion data based on the glenohumeral joint comprises translation data of the humerus and the scapula relative to one another for the population of persons or
the motion data comprise rotation trajectories of the humerus and the scapula relative to one another for the population of persons.

7. The method of claim 6, wherein determining track engagement comprises determining a glenoid track virtual object based on the one or more 3D models.

8. The method of claim 7, wherein characteristics of the glenoid track virtual object comprise one or more of:
a glenoid track length;
a glenoid track distance;
a glenoid track area;
a glenoid track angle; and
combinations thereof.

9. The method of any preceding claim, wherein the motion data based on the glenohumeral joint comprises a terminal position of the humerus relative to the scapula.

10. The method of claim 9, wherein the terminal position of the humerus relative to the scapula is further defined as a maximum position of translation data of the humerus relative to the scapula, or wherein the terminal position of the humerus relative to the scapula is further defined as a maximum position of rotation data of the humerus relative to the scapula.

11. The method of claim 9 or 10, wherein determining track engagement comprises determining a glenoid track virtual object based on the terminal position of the humerus relative to the scapula, further comprising displaying the glenoid track virtual object relative to a portion of a rendering of the one or more 3D models.

12. The method of claim 9, 10 or 11, further comprising displaying a rendering of the one or more 3D models in the terminal position.

13. The method of any one of claims 9-12, further comprising determining a glenoid projection virtual object based on the terminal position of the humerus relative to the scapula; and identifying a lesion virtual object on a humeral head portion of humerus 3D model.

14. The method of claim 13, wherein the one or more 3D models comprises a humerus 3D model and a scapula 3D model, wherein determining the track engagement comprises determining a characteristic based on a first area of the glenoid projection virtual object and a second area of the lesion virtual object.

15. The method of claim 9, or any of claims 10-14 as far as dependent from claim 9, wherein the motion data comprises an alternate position of the humerus relative to the scapula other than the terminal position, the alternate position corresponding to a different position of the translation and rotation data than the terminal position, wherein the one or more 3D models comprises a humerus 3D model and a scapula 3D model, wherein determining track engagement comprises determining a glenoid track virtual object based on the terminal position and the alternate position on the humerus 3D model and the scapula 3D model, and wherein the method further comprises displaying a rendering of the humerus 3D model and the scapula 3D model in the alternate position and the terminal position.

16. The method of claim 15, wherein the one or more 3D models comprises a humerus 3D model and a scapula 3D model, wherein applying motion data to the humerus 3D model and the scapula 3D model further comprises determining a margin of deviation based on the terminal position and the alternate position of the humerus 3D model and the scapula 3D model and a terminal position and an alternate position of a humerus and a scapula of one or more persons from the population of persons.

17. A computing system comprising: a memory configured to store image data of a patient shoulder anatomy; and
a controller configured to perform the method of any of claims 1-16.
